# EUROPEAN PATENT APPLICATION

(11) **EP 4 744 578 A1**
(43) Date of publication of application: **20.05.2026**
(21) Application number: 24306936.6
(22) Date of filing: 19.11.2024
(51) Int. Cl.: A61B 5/00, A61B 5/024, A61B 5/08, A61B 5/11

(54) **EXTRACTING VITAL SIGNALS FROM PRESSURE SENSOR DATA**

(71) Applicant: Koninklijke Philips N.V., 5656 AE Eindhoven (NL)
(72) Inventor: WIEMKER, Rafael, 5656 Eindhoven (NL); DENISSEN, Ad, 5656 Eindhoven (NL); VAN EE, Raymond, 5656 Eindhoven (NL); KRUEGER, Sascha, 5656 Eindhoven (NL); SENEGAS, Julien, 5656 Eindhoven (NL); WIRTZ, Daniel, 5656 Eindhoven (NL)
(74) Representative: Philips Intellectual Property & Standards

(57) **Abstract**

Disclosed herein is a method for extracting cardiac signal data (428) from a set of sensor data (412) acquired using a plurality of pressure sensors (102) of a two-dimensional sensor array (100). The method comprises receiving (200) the set of sensor data (412), determining (202) a principal respiratory time signal (342; 416) of a respiratory motion pattern for the subject (118), determining (204) sensor-wise individual phase-shifts (418) of individual respiratory time signals, eliminating (208) the determined principal respiratory time signal (342; 416) sensor-wise using the determined individual phase-shifts (418) for the individual sensors (102), determining (10) a principal cardiac time signal (424) of a cardiac motion pattern for the subject (118), and outputting (216) cardiac signal data (428) comprising the principal cardiac time signal (424).

## Description

### FIELD OF THE INVENTION

The invention relates to the field of vital signal acquisition, in particular it relates to an extracting of cardiac signal data from a set of sensor data acquired using a plurality of pressure sensors of a two-dimensional sensor array.

### BACKGROUND OF THE INVENTION

Two-dimensional pressure sensor arrays, in particular two-dimensional pressure sensor arrays configured for a subject to lie thereon, may be suitable for measuring and monitoring specific locations and motions of subjects arranged thereon. A two-dimensional distribution of pressure sensors may be able to detect a two-dimensional distribution of pressure changes over time caused by motions of a subject arranged thereon. However, an extracting of data signals descriptive of specific motions, e.g., of vital signals, may be challenging, since the pressure sensors are only able to detect a superposition of all simultaneous motions of the subject.

### SUMMARY OF THE INVENTION

The invention provides for a method for extracting cardiac signal data from a set of sensor data acquired using a plurality of pressure sensors of a two-dimensional sensor array in the independent claims. The invention further provides for a computer program as well as a medical system comprising a computational system for extracting cardiac signal data from a set of sensor data acquired using a plurality of pressure sensors of a two-dimensional sensor array in the independent claims. Exemplary embodiments are given in the dependent claims.

In one aspect, a method for extracting cardiac signal data from a set of sensor data acquired using a plurality of pressure sensors of a two-dimensional sensor array is disclosed. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

The method comprises receiving the set of sensor data. A principal respiratory time signal of a respiratory motion pattern for the subject is determined using the set of sensor data. The determined principal respiratory time signal is a common one-dimensional respiratory time signal for the plurality of sensors. Sensor-wise individual phase-shifts of individual respiratory time signals comprised by the individual datasets of the individual sensors are determined. The determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. A principal cardiac time signal of a cardiac motion pattern for the subject is determined using the set of residual sensor data. The determined principal cardiac time signal is a common one-dimensional cardiac time signal for the plurality of sensors. Cardiac signal data comprising the principal cardiac time signal is output.

In another aspect, a computer program for extracting cardiac signal data from a set of sensor data acquired using a plurality of pressure sensors of a two-dimensional sensor array is disclosed. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

The computer program comprises machine executable instructions for execution by a computational system. Execution of the machine executable instructions causes the computational system to receive the set of sensor data. A principal respiratory time signal of a respiratory motion pattern for the subject is determined using the set of sensor data. The determined principal respiratory time signal is a common one-dimensional respiratory time signal for the plurality of sensors. Sensor-wise individual phase-shifts of individual respiratory time signals comprised by the individual datasets of the individual sensors are determined. The determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. A principal cardiac time signal of a cardiac motion pattern for the subject is determined using the set of residual sensor data. The determined principal cardiac time signal is a common one-dimensional cardiac time signal for the plurality of sensors. Cardiac signal data comprising the principal cardiac time signal is output.

In another aspect, a medical system comprising a computational system for extracting cardiac signal data from a set of sensor data acquired using a plurality of pressure sensors of a two-dimensional sensor array is disclosed. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

The computational system comprises a memory storing machine executable instructions. Execution of the machine executable instructions causes the computational system to receive the set of sensor data. A principal respiratory time signal of a respiratory motion pattern for the subject is determined using the set of sensor data. The determined principal respiratory time signal is a common one-dimensional respiratory time signal for the plurality of sensors. Sensor-wise individual phase-shifts of individual respiratory time signals comprised by the individual datasets of the individual sensors are determined. The determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. A principal cardiac time signal of a cardiac motion pattern for the subject is determined using the set of residual sensor data. The determined principal cardiac time signal is a common one-dimensional cardiac time signal for the plurality of sensors. Cardiac signal data comprising the principal cardiac time signal is output.

### BRIEF DESCRIPTION OF THE DRAWINGS

In the following preferred embodiments of the invention will be described, by way of example only, and with reference to the drawings in which:
Fig. 1 shows a flow chart illustrating an exemplary method for extracting cardiac signal data;
Fig. 2 shows a flow chart illustrating another exemplary method for extracting cardiac signal data;
Fig. 3 shows a flow chart illustrating an exemplary method for extracting cardiac and respiratory signal data;
Fig. 4 shows a flow chart illustrating another exemplary method for extracting cardiac and respiratory signal data;
Fig. 5 shows a flow chart illustrating another exemplary method for extracting cardiac signal data;
Fig. 6 shows a flow chart illustrating an exemplary method for determining a principal respiratory time signal;
Fig. 7 shows a flow chart illustrating an exemplary method for determining a principal cardiac time signal;
Fig. 8 shows an exemplary two-dimensional sensor array with a plurality of pressure sensors;
Fig. 9 shows an exemplary respiratory signal correlation map;
Fig. 10 shows an exemplary respiratory signal covariance map;
Fig. 11 shows an exemplary respiratory signal phase-shift map;
Fig. 12 shows an exemplary map comprising correlation and covariance information;
Fig. 13 shows an exemplary principal respiratory time signal;
Fig. 14 shows an exemplary computational system of an exemplary medical system;
Fig. 15 shows an exemplary medical system;
Fig. 16 shows an exemplary medical system comprising an exemplary medical imaging system in form of an MRI system; and
Fig. 17 shows an exemplary medical system comprising an exemplary medical imaging system in form of a CT system.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In an example, a method for extracting cardiac signal data from a set of sensor data acquired using a plurality of pressure sensors of a two-dimensional sensor array is provided. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

The method comprises receiving the set of sensor data. A principal respiratory time signal of a respiratory motion pattern for the subject is determined using the set of sensor data. The determined principal respiratory time signal is a common one-dimensional respiratory time signal for the plurality of sensors. Sensor-wise individual phase-shifts of individual respiratory time signals comprised by the individual datasets of the individual sensors are determined. The determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. A principal cardiac time signal of a cardiac motion pattern for the subject is determined using the set of residual sensor data. The determined principal cardiac time signal is a common one-dimensional cardiac time signal for the plurality of sensors. Cardiac signal data comprising the principal cardiac time signal is output.

Examples may have the beneficial effect, that they enable a cardiac time signal extraction from a two-dimensional sensor array of pressure sensors. The two-dimensional sensor array may, e.g., be embedded in a mattress. Thus, a pressure-sensing mattress may be provided. Examples may have the beneficial effect, that a two-dimensional sensor array of pressure sensors may, e.g., be inexpensive to manufactured and simple to use without requiring a dedicated preparation. At the same time, examples may allow for an extraction of a precise cardiac time signal. Such a cardiac time signal may, e.g., be used for monitoring a vital state of the subject and/or for triggering a medical imaging data acquisition. The triggering may, e.g., be configured to acquire the medical imaging data at a certain point of time of a cardiac cycle of the subject. Examples may in addition be able to extract a respiratory time signal, e.g., a principle respiratory time signal, from the two-dimensional sensor array of pressure sensors, i.e., from the plurality of individual sensor datasets with the sensor data acquired by the individual pressure sensors of the sensor array. Using sensor data acquired by a plurality of pressure sensors may, e.g., allow for an extraction of a respiratory time signal, which is more precise than a respiratory time signal extracted from sensor data provided by a single pressure sensor only. A precision of the respiratory time signal being extracted may, e.g., be further increased by using further processing measures as described in the following, like a filtering of the sensor data, a smoothening of the respiratory time signal, performing a phase-correction for the respiratory time signal, etc. Such a respiratory time signal may, e.g., be used for monitoring a vital state of the subject and/or for triggering a medical imaging data acquisition. The triggering may, e.g., be configured to acquire the medical imaging data at a certain point of time of a respiratory cycle of the subject and/or to acquire the medical imaging data at a state of minimum or even no breathing related movement of the subject. The later may, e.g., reduce or even prevent motion artifacts. Furthermore, respiratory time signal may, e.g., be used to determine breathing related movements of body parts of the subject. This may, e.g., support a focusing of imaging data acquisition on certain body parts by enabling a precise breathing dependent position determining and/or predicting of the same.

Medical imaging refers to a method of acquiring medical imaging data, i.e., interior or exterior imaging data of a subject, which may, e.g., be used or clinical analysis. Medical imaging methods may, e.g., comprise such methods as radiography, magnetic resonance imaging, ultrasound, computed tomography, etc.

Examples describe a specific algorithmic scheme to process a stream of incoming pressure sensor array time signals from a plurality of pressure sensors of a two-dimensional sensor array to yield simultaneously a signal curve, in particular a real-time signal curve, for a cardiac cycle. Furthermore, the scheme may also be able to yield in addition a signal curve, in particular a real-time signal curve, for a respiratory cycle. Example may have the beneficial effect, that for the determining of the respective signal curves two-dimensional sensor data is used.

Such a non-trivial, integrated signal processing scheme for reconstruction cardiac signal data may comprise a reconstruction of respiratory signal data. Both signals, i.e., cardiac and respiratory signal, are spatially and timely intertwined and fall within adjacent or even overlapping frequency ranges, but have different orders of magnitude in amplitude. Examples may have the beneficial effect of using the respiratory signal data, which has a larger amplitude compared to the cardiac signal, for the reconstruction of the cardiac signal data. Thus, the reconstruction of the cardiac signal data may be improved. In particular, a two-dimensional distribution of the respiratory signal data, e.g., in form of the phase-shifts of the individual respiratory time signals of the individual sensors, may be used for the reconstruction of the cardiac signal data. Using a two-dimensional distribution of the respiratory signal data for the reconstruction of the cardiac signal data may enable a precise reconstruction of the cardiac signal data. This may in particular be true for a reconstruction of a two-dimensional representation of the cardiac signal data.

Using reconstructed respiratory signal data for the reconstruction of the cardiac signal data may not only improve the reconstruction of the cardiac signal data, but may in addition enable a provision of both signal curves for cardiac as well as respiratory signal data. The signal curves may in particular be provided simultaneously. Examples may have the beneficial effect, that both signal data, i.e., cardiac signal data and respiratory signal data may be determined using the same sensors, i.e., the pressure sensors of the two-dimensional sensor array.

A one-dimensional time signal refers to a signal, which describes a variation of a scalar quantity to be measured over time. The one-dimensional time signal may, e.g., be provided in form of a set of values of the quantity to be measured, e.g., a pressure, acquired at different points of time. The values describe the variation of the quantity to be measured over time.

The individual pressure sensors of the two-dimensional sensor array may be configured to detect local pressure changes over time. Thus, each of the individual sensors may acquire sensor data, which quantifies a value of a local pressure at the position of the respective pressure sensor within the two-dimensional sensor array over time. The sensor data of an individual sensor dataset of an individual pressure sensor may therefore comprise a plurality of pressure values acquired for different points in time. These pressure values describe the local pressure at the position of the respective pressure sensor over time as a one-dimensional function of pressure over time, i.e., a one-dimensional time signal of the local pressure.

The one-dimensional functions of pressure over time of the individual pressure sensors of the plurality of pressure sensors may, e.g., be combined with each other, e.g., averaged, providing a single common one-dimensional function of pressure over time, i.e., a single common one-dimensional time signal of pressure for the plurality of pressure sensors.

For providing a two-dimensional representation of the pressure, e.g., a plurality of two-dimensional maps of the pressure values acquired by the individual pressure sensors may be provided. Each of the maps may, e.g., describe for a different point in time the individual pressure values at the different positions of the individual pressure sensors. Thus, a two-dimensional distribution of the pressure per point in time may be provided.

For providing a two-dimensional representation of the pressure, e.g., the single common one-dimensional time signal may be used and correlation values, covariance values, and/or phase-shifts for the individual one-dimensional time signals of the local pressures at the positions of the individual pressure sensors with respect to the single common one-dimensional time signal may be determined. The result of this determination, may, e.g., be provided in form of a correlation map, a covariance map, and/or a phase-shift map. Since for the determining of the single correlation values, covariance values, and/or phase-shifts one dimensional functions over time are used rather than values for a single point in time, there is a single correlation map, covariance map, and phase-shift map for the whole time span, over which the one-dimensional functions are taken into account.

For example, the sensor data of the individual sensor datasets may comprise individual respiratory time signals, i.e., one-dimensional time signals of the local pressures descriptive of local variations of the pressure due to local respiratory motion patterns. These individual respiratory time signals may, e.g., be combined with each other to provide a principal respiratory time signal of an overall respiratory motion pattern with the determined principal respiratory time signal being a common one-dimensional respiratory time signal for the plurality of sensors. For example, further a two-dimensional representation of the pressure due to the local respiratory motion patterns may be provided as described above for a pressure in general.

For example, the residual sensor data of the individual residual sensor datasets may comprise individual cardiac time signals, i.e., one-dimensional time signals of the local pressures descriptive of local variations of the pressure due to local cardiac motion patterns. These individual cardiac time signals may, e.g., be combined with each other to provide a principal cardiac time signal of an overall cardiac motion pattern with the determined principal cardiac time signal being a common one-dimensional cardiac time signal for the plurality of sensors. For example, further a two-dimensional representation of the pressure due to the local cardiac motion patterns may be provided as described above for a pressure in general.

Specifically, the method for extracting cardiac signal data may foresee as a key element a determining of a principal respiratory signal, which is used to determine residual signal data, from which a principal cardiac signal is extracted. Thus, the method may enable an extraction of a principal respiratory signal in addition to the principal cardiac signal. The principal respiratory signal may be provided as part of the extraction of the principal cardiac signal. In addition, e.g., one or more spatial mappings of cardiac signal data over the two-dimensional sensor array extent, e.g., a mattress extent, may be determined. In addition, e.g., one or more spatial mappings of respiratory signal data over the two-dimensional sensor array extent, e.g., a mattress extent, may be determined.

Thus, example may provide a spatio-temporal signal processing method for extracting a cardiac signal from a two-dimensional array of pressure sensors, e.g., embedded in a mattress, on which a subject is lying, whereby a spatial manifestations of signal correlations of respiratory and cardiac signals are used to improve the results. Example may, e.g., provide a spatio-temporal signal processing method for extracting both cardiac and respiratory signals from a two-dimensional array of pressure sensors, e.g., embedded in a mattress, on which a subject is lying.

Examples of the method may comprise determining a principal respiratory time signal using a fusion, e.g., a time-lag-adjusted aggregation, of the sensor data acquired by the individual sensors of the sensor array, sensor-wise eliminating of the principal respiratory signal using, e.g., a respiratory phase-shift mapping, yielding a set of residual sensor data comprising a plurality of individual residual sensor datasets with residual sensor data for the individual sensors, and determining a principal cardiac time signal using a fusion, e.g., a time-lag-adjusted aggregation, of the of the residual sensor data of the individual sensors. Examples may, e.g., further comprise determining two-dimensional respiratory spatial mappings, in particular a correlation map, a covariance map, and/or a phase-shift map. Examples may, e.g., further comprise determining two-dimensional cardiac spatial mappings, in particular a correlation map, a covariance map, and/or a phase-shift map. Examples may, e.g., further comprise determining two-dimensional respiratory-cardiac spatial mappings. Such respiratory-cardiac spatial mappings may, e.g., be used for a visualization of a spatially resolved interaction between both vital signals, i.e., cardiac signal and respiratory signal.

An extraction of a cardiac and/or respiratory time signal from a two-dimensional array of pressure sensors may, e.g., reduce or even avoid a need for additional sensors, such as ECG-leads and/or breathing-belts or breathing-bellows. A two-dimensional array of pressure sensors with a plurality of pressure sensors may, e.g., be embedded in a mattress, in particular in a CT- and/or MRI-compatible mattress.

A current heart rate and/or breathing rate of a subject and/or phase time points for these rates may be beneficial or even necessary control parameters for certain medical imaging methods, e.g., a computed tomography imaging, magnetic resonance imaging, positron emission tomography imaging, and/or ultrasound imaging. These control parameters may, e.g., be used to mitigate motion artefacts and/or time an imaging data acquisition to be executed at a certain state of a physiological cycle, such as a cardiac cycle and/or a breathing cycle.

In some types of imaging methods, e.g., some types of CT- or MR imaging methods, an acquisition of imaging data needs to be orchestrated with a heart rhythm, e.g., rate and/or phase, and/or a breathing rhythm, e.g., rate and/or phase, of a subject. Such an orchestration may, e.g., be required for reducing motion artefacts in resulting images. This may specifically be the case for an imaging of a heart, of coronaries, of pulmonary airways, of an abdomen, and/or for images, which are intended to be used for a tumor tracking. In imaging methods, which are used for imaging a contrast agent, a propagation of the contrast agent may depend on the heart rate. Thus, a timing of imaging data acquisition may require insight into the respective heart rate, e.g., in order to be able to estimate a state of propagation of the contrast agent.

The two-dimensional sensor array may, e.g., be embedded into a mattress. The respective mattress may, e.g., be a mattress-like thin blanket comprising the two-dimensional sensor array. The mattress may, e.g., be configured to be placed underneath a subject, such that the respective subject may, e.g., lay down on to the mattress with the two-dimensional sensor array. The two-dimensional sensor array and/or mattress may, e.g., be arranged on a subject support, e.g., a subject support of a medical imaging system. A subject support, e.g., refers to a part of a medical imaging system, which is configured to support a subject, e.g., an examination table.

The two-dimensional sensor array may, e.g., be a regular array of pressure sensors. The pressure sensors of the two-dimensional sensor array may, e.g., be pressure sensors of the same sensor type. The two-dimensional sensor array may, e.g., comprise pressure sensors of different sensor types. The pressure sensors of the two-dimensional sensor array may, e.g., comprise fiber-optical pressure sensors with fiber crossings, fiber-optical pressure sensors with fiber Bragg gratings, piezo-electrical pressure sensors, capacitive pressure sensors, and/or gas/air pocket/bellow pressure sensors.

For example, fiber-optical pressure sensors with fiber crossings may use crossing fibers for providing pressure sensors. A crosspoint defining a pressure sensor may, e.g., comprise two fibers in contact with each other at an intersection of the same. An amount of coupled light between the two fibers may, e.g., dependent on a pressure applied to the crosspoint, which may thereby act as a pressure sensor.

For example, fiber-optical pressure sensors with fiber Bragg gratings may use the fiber Bragg gratings for providing pressure sensors. A fiber Bragg grating (FBG) is a type of distributed Bragg reflector constructed in a short segment of optical fiber that reflects particular wavelengths of light, while transmits all other wavelengths of light. This feature may be achieved by creating a periodic variation in the refractive index of the fiber core, which generates a wavelength-specific dielectric mirror. During a pressure change, a wavelength of the reflected light is being affected due to a change of fiber birefringence. Thus, a fiber Bragg grating may be used as a pressure sensor.

Using fiber-optical pressure sensors may, e.g., have the beneficial effect, that they may be suitable for usage inside a gantry of a medical imaging system, such as a CT imaging system and/or an MR imaging system, without causing imaging artefacts. Fiber-optical pressure sensors may, e.g., comprise no metal parts and/or electrical wiring, thereby avoiding causing imaging artefacts, e.g., in CT imaging and/or an MRI. Especially, MRI and/or CT types of medical imaging may suffer from limitation regarding the type of pressure sensors, which might be suitable for usage such medical imaging methods. Fiber-optical pressure sensors may, e.g., be immune and non-disturbing with respect to strong magnetic and/or radio frequency fields used for acquiring MRI data. The fiber-optical pressure sensors comprised by the two-dimensional sensor array may, e.g., be arranged on the subject support or table, which is arranged within the gantry for imaging data acquisition.

Examples may have the beneficial effect, that a spatial mapping of the respiratory signal data in form of the determined sensor-wise individual phase-shifts of the individual respiratory time signals comprised by the individual datasets of the individual sensors is used for the determining of the principal cardiac time signal. Thus, a better signal separation by utilizing the spatial manifestations of the signals' correlations, i.e., of the respiratory signal data and the cardiac signal data may be obtained. Furthermore, examples may also enable a spatial mapping of both signals, i.e., of the respiratory signal data and the cardiac signal data, over the two-dimensional sensor array.

Examples may have the beneficial effect of using a two-dimensional distribution of the respiratory signal data, e.g., in form of the phase-shifts of the individual respiratory time signals of the individual sensors. Using two-dimensional distribution of the respiratory signal data may be beneficial compared to, e.g., a single-point respiratory signal, which may, e.g., be acquired using a belt or air bellow and may not be sufficiently descriptive to reflect various breathing-related body motions required for motion-blur-free imaging.

Examples may have the beneficial effect, that, contrary to using, e.g., an electrocardiography device (ECG) with leads, for using a two-dimensional sensor array, which is configured for a subject to lay down thereon, e.g., no tedious preparation, no experienced technician for lead placing, no shaving of chest hair, and no touching of the subject may be required.

Examples may have the beneficial effect, that sensor data acquired by the pressure sensors may not be degraded by a medical imaging method used to acquire medical imaging data. In contrast, e.g., ECG signals may be corrupted by a magnetohydrodynamic (MHD) effect during an MR imaging. The MHD effect refers to a comparably small voltage, which is generated by ionic currents in descending aorta. This voltage may result in field-strength dependent ECG changes, especially in elevated T-waves. The MHD effect may especially occur at field strengths equal to or larger than 3T, but may already occur at lower field strength, e.g., in a range from 1T to 3T and/or even below 1T.

In a magnetic field of a magnetic resonance imaging system, e.g., an elevation of a T-wave of an ECG signal may occur. This elevation may be so marked that the T-wave may, e.g., become larger than a QRS-complex of the ECG signal, i.e., a combination of graphical deflections in form of Q, R, and S waves comprised by a typical ECG signal, which are caused by a depolarization of the right and left ventricles of a heart and contraction of large ventricular muscles. Furthermore, a reduction of the R-wave may also occur. The R- wave may even be inverted occasionally. The MHD effect, i.e., an induction of a voltage in a conductive fluid flowing through a magnetic field, in this case results from a superimposed voltage within blood in the descending thoracic aorta, which is been induced by the bloods' flow in the magnetic field. In humans imaged at 1.5 T, the MHD-induced voltage may, e.g., be on the order of 5 mV to 10 mV and may be even larger at 3.0 T and above. The reason for this voltage to be superimposed on the T-wave is that this is the time of maximum flow in the descending aorta.

Also, in medical imaging method, like in CT systems, an ECG signal may, e.g., be degraded.

Examples may have the beneficial effect of providing a method for extracting cardiac signal data independently of a specific location of the subject lying on the two-dimensional sensor array. The subject may be positioned at an arbitrary location on the two-dimensional sensor array, e.g., provided embedded into a pressure-sensing-mattress, such that an arbitrary combination of pressure sensors of the two-dimensional sensor array may detect a pressure change. Examples may enable an extraction of cardiac signal data from a set of sensor data descriptive of pressure changes detected over time by such an arbitrary combination of individual sensors of the sensor array due to motions of a subject positioned at such an arbitrary location on the two-dimensional sensor array.

Examples may have the beneficial effect of enabling a handling of changes of respiratory and/or cardiac related motion patterns over time. Thus, examples may not only be able to handle stationary motion patterns.

Examples may have the beneficial effect of providing a method capable of handling incoming sensor data from the plurality of pressure sensors of the sensor array, which may yield a high data volume, e.g., a M × N × T tensor. There may, e.g., be M × N pressure sensors comprised by the two-dimensional sensor array with M and N being natural numbers greater than 1 and data may, e.g., be acquired over a time window of T timepoints with T being a natural number greater than 1. The sensor data may, e.g., be a superposition of sensor data descriptive of body, respiratory, and/or cardiac motion patterns.

Examples may have the beneficial effect of providing a method which enables an effective separation of the respiratory and cardiac sensor data. Even during rest-periods, in which the subject lies still, a respiratory vs cardiac source separation may be challenging as a cardiac motion may reach the pressure sensors of the two-dimensional sensor array with, e.g., as much as two orders of magnitude less signal power compared to a respiratory motion of the subject. In addition, cardiac and respiratory may, e.g., spatially overlap, partially or even entirely, within respiratory-driven body parts of the subject. Examples may be able to handle such a superposition of spatially overlapping signals with different amplitudes.

Examples may be specifically designed to extract cardiac signal data from a set of sensor data comprising superimposed cardiac and respiratory sensor data. Examples may be specifically designed to extract cardiac signal data from a set of sensor data acquired using a plurality of pressure sensors of a two-dimensional sensor array and comprising a plurality of individual sensor datasets with sensor data descriptive of pressure changes detected over time by individual pressure sensors due to motions of a subject lying on the sensor array.

Examples may in particular be configured to cope with the following conjunction of challenges: Typical respiratory and cardiac frequency ranges are comparatively close and may even overlap. A typical frequency range for a heart rate may, e.g., be 0.8 Hz to 2.2 Hz, i.e., 50 BPM to 130 BPM, while a typical frequency range for a breathing rate may, e.g., be 0.15 Hz to 1.0 Hz. Examples may be able to separate such signals with close or at least partially overlapping frequencies. Furthermore, both rates, i.e., heart rate and breathing rate, may, e.g., change quickly. Contrary to algorithms, which rely on long observation intervals to deal with high noise, examples may be able to handle such quickly changing rates, since no long observation intervals are required. Furthermore, respiratory and cardiac pressure signal amplitudes are orders of magnitude different in signal power. Contrary to algorithms, which are designed for similar amplitudes, like, e.g., an independent component analysis (ICA), examples may be able to handle such signals with an order of magnitudes difference in signal power. Furthermore, respiratory and cardiac pressure signals may not be distinguishable by directional sensing, as they come from the same body region. In addition, an original cardiac signal is further dispersed after propagation though the body on its path to the underlying sensor array, e.g., embedded into a mattress. Examples may not rely on a directional sensing and therefore be able to separate respiratory and cardiac pressure signals coming from the same body region.

Examples may, e.g., use an averaging of FFTs from a plurality of different spatial locations, i.e., from a plurality of different pressure sensors of the two-dimensional sensor array. In other words, a spatial reinforcement of a weak noisy signal, e.g., the cardiac time signal, rather than a temporal reinforcement may be used. Thus, examples may, e.g., be able to handle quick changes of the signal in time.

Examples may, e.g., enable a better signal separation by utilizing a spatial distribution of individual sensor signal correlations, rather than simply filtering out confounding motion and respiratory signals by applying simple frequency filters.

Examples comprise determining a respiratory signal as a principal signal and determining specific variation of the respiratory signal per sensor, in order to eliminate the respiratory signal from the overall mixed signals and to determine a weaker cardiac signal from the resulting residual signals per sensors. These cardiac signals per sensor are used to determine a principal cardiac signal. In addition, e.g., a refined sensor-wise cardiac signal may determine. In addition, the spatially resolved respiratory and cardiac signals may, e.g., be correlated for further insights.

Examples may determine, e.g., principal time signals, i.e., 1D time curves, for cardiac and/or respiratory motions as well as spatially resolved maps for the cardiac and/or respiratory motions. These spatially resolved maps may, e.g., be provided in form of a 4D tensor with stacked maps. The first two dimensions may, e.g., be the spatial dimensions of the two-dimensional sensor array, a third dimensional may be time and a fourth dimension may be provided by the two possible types of data, i.e., respiratory and cardiac signal data.

Cardiac and/or respiratory signal data extracted according to examples may, e.g., be used to assist with a medical imaging. Cardiac and/or respiratory signal data extracted according to examples may, e.g., be used to assist with a medical imaging using a gantry-based imaging system, like a CT system or an MRI system. Cardiac and/or respiratory signal data extracted according to examples may, e.g., be used to assist with a medical imaging using ultrasound. Cardiac and/or respiratory signal data extracted according to examples may, e.g., be used to assist with a subject monitoring, e.g., an emergency monitoring, a stationary bed-side monitoring, and/or monitoring in a neonatal intensive care unit (NICU).

The determining of the principal respiratory time signal is, e.g., carried out using sensor data of a first time window, e.g., 20 s, before a reference point of time, e.g., a current point of time or a point of time selected in retrospect. For example, the set of sensor data being received comprises sensor data acquired during the respective first time window. For example, sensor data acquired during the respective first time window is selected from the received set of sensor data for further processing.

The determining of the principal cardiac time signal, e.g., comprises selecting residual sensor data of a second time window, e.g., 5 s, before a reference point of time, e.g., a current point of time. For example, the first time window is shorter than the second time window. For example, the first and second reference points of time are identical. For example, the selection is carried out for each individual sensor of the two-dimensional sensor array, i.e., for each individual residual sensor dataset.

Pressure changes described by the sensor data acquired using the plurality of pressure sensors may, e.g., be descriptive of cardiac activities of the subject. The output cardiac signal data may, e.g., be used to detect, monitor and/or predict cadiac motion patterns of the subject. Pressure changes described by the sensor data acquired using the plurality of pressure sensors may, e.g., be descriptive of respiratory activities of the subject. Additionaly output respiratory signal data may, e.g., be used to detect, monitor and/or predict respiratory motion patterns of the subject. The sensor data may, e.g., be used to determine cadiac motion patterns and respiratory motion patterns simultaneously. Pressure changes described by the sensor data acquired using the plurality of pressure sensors may, e.g., be descriptive of respiratory-cardiac activities of the subject. Output cardiac and respiratory signal data may, e.g., be used to detect, monitor and/or predict cadiac motion patterns of the subject.

A medical imaging method may, e.g., comprise a tomographic scan, such as an MRI or CT scan. The method may, e.g., further comprise using the determined cardiac and/or respiratory motion a for cardio and/or respiratory timing of the tomographic scan, i.e., of the medical imaging data acquisition.

A cardiac signal, like a principal cardiac time signal, may, e.g., be used for a cardiac triggering a medical imaging data acquisition using a medical imaging system, like a magnetic resonance imaging data acquisition using a magnetic resonance imaging system.

Using a cardiac triggering for a magnetic resonance imaging data acquisition may have the beneficial effect that, e.g., effects of a cardiac related pulsating of the brain and/or of a cardiac related pulsatile of cerebrospinal fluid CSF may be compensated. For example, cardiac related negative effects on a diffusion-weighted magnetic resonance imaging (DWI) may be compensated. DWI refers to a specific approach, in which magnetic resonance images are generated from data that uses a diffusion of water molecules to generate contrast in magnetic resonance images. DWI allows a mapping of a diffusion process of molecules, mainly water, in biological tissues, in vivo, and/or non-invasively. This molecular diffusion may reflect interactions with obstacles, such as, e.g., macromolecules, fibers, and membranes. Water molecule diffusion patterns may reveal microscopic details about tissue architecture, either normal or in a diseased state. A special kind of DWI is referred to as diffusion tensor imaging (DTI), which may be used to map white matter tractography in the brain.

Outputting a live cardiac signal of the subject with at least a heart rate enabling, e.g., a monitoring of the heart rate. Such a cardiac signal may, e.g., provide for subject monitoring and may, e.g., allow to observe whether the subject becomes uncomfortable or nervous during a medical imaging data acquisition.

Determining respiratory motion patterns of a subject may, e.g., comprise determining respiratory motion patterns of the subject at multiple locations. The multiple locations may comprise locations in the chest and/or abdomen of the subject. The method may further comprise using the determined respiratory motion patterns to detected, monitored and/or predicted a motion of one or more patient organs.

For example, the determining of the principal respiratory time signal further comprises a filtering of the sensor data comprised by the set of sensor data. The filtering comprises a selecting of signal data from a predefined first frequency range of respiratory signal frequencies.

In an optional pre-processing step, confounding non-respiratory signals comprised by the sensor data may be filtered out. For example, a frequency analysis using a Fortier transform may be performed. For example, only if a one-dimensional time signal has a strong frequency amplitude in an appropriate frequency range, i.e., the predefined first frequency range of respiratory signal frequencies, the respective one-dimensional time signal may be used for determining the principal respiratory time signal. For example, only if a one-dimensional time signal has a strong frequency amplitude in the appropriate frequency range, the respective one-dimensional time signal is added to the principal signal. The predefined first frequency range of respiratory signal frequencies is, e.g., a frequency range from 0.15 Hz to 1.0 Hz.

For example, the determining of the principal respiratory time signal further comprises an adding up of the individual respiratory time signals comprised by the individual datasets of the individual sensors. The individual respiratory time signals of the individual sensors may, e.g., be added up point-wise per point of time.

For example, the determining of the principal respiratory time signal further comprises an averaging over the individual respiratory time signals comprised by the individual datasets of the individual sensors. The principal respiratory time signal may be a one-dimensional time signal. The determining of the principal respiratory time signal may, e.g., comprise a point-wise, i.e., per point in time, adding up of individual respiratory time signals of the individual sensors. The adding up may, e.g., be comprised by the averaging over the individual respiratory time signals.

For example, the determining of the principal respiratory time signal further comprises a determining an eigenvector corresponding to a largest eigenvalue of the individual respiratory time signals comprised by the individual datasets of the individual sensors.

The two-dimensional senor array may, e.g., comprise N sensors. The N individual respiratory time signals of the N sensors may, e.g., comprise T time points each, i.e., T sample values for T time points. The N individual respiratory time signals of the N sensors with the T time points each may, e.g., be assembled into an N × T matrix. For example, a symmetric T × T covariance matrix may be determined for the N × T matrix. For this T × T covariance matrix a singular value may be computed or an eigenvalue matrix decomposition may be performed. A most significant eigenvector, i.e., an eigenvector corresponding to a largest eigenvalue, may be determined as the principal vector, i.e., as the principal respiratory time signal.

Examples may have the beneficial effect, that a risk of contradicting signals from the various sensors of the two-dimensional sensor array are cancelling out completely may be retired.

For example, the determining of the principal respiratory time signal further comprises a successive averaging of selected individual respiratory time signals comprised by the individual datasets of the individual sensors using a covariance analysis for the selecting. The successive averaging starts with averaging of a pair of individual respiratory time signals, for which a highest covariance compared to all other pairs of individual respiratory time signals is determined.

Each further step of the successive averaging comprises, until a termination criterion is satisfied, determining covariances of a current averaged respiratory time signal and remaining individual respiratory time signals, selecting a remaining individual respiratory time signal, for which a highest covariance with respect to the current averaged respiratory time signal is determined compared to all other remaining individual respiratory time signals, and averaging the current averaged respiratory time signal and the selected remaining individual respiratory time signal.

All time series of all sensors may be pairwise evaluated by an absolute magnitude of their mutual covariance. In an iterative scheme, starting with the highest covariance pair, more and more time series of other sensors of the plurality of sensors may be added. The iteration may be stopped, when an overall total covariance and/or an overall total correlation is no longer increasing. Stopping the iteration, when the overall total covariance and/or the overall total correlation is no longer increasing, may prevent an adoption of outliers for the determining of the principal respiratory time signal.

Covariance is a measure of the joint variability of two random variables or sets of data, e.g., of two respiratory time signals. A sign of the covariance shows a tendency in a linear relationship between the respective variables. If greater values of one variable mainly correspond with greater values of the other variable, while lesser values of one variable mainly correspond with lesser values of the other variable, i.e., if both variables tend to show similar behavior, the covariance is positive. In the opposite case, when greater values of one variable mainly correspond to lesser values of the other, i.e., if the variables tend to show opposite behavior, the covariance is negative. The magnitude of the covariance is the geometric mean of the variances that are in common for the two random variables.

Alternatively to a covariance, e.g., a correlation coefficient may be used. The correlation coefficient, also referred to as Pearson correlation coefficient, measures linear correlation between two sets of data. It is the ratio between the covariance of two variables and the product of their standard deviations. It is essentially a normalized measurement of the covariance, such that the result has a value between -1 and 1. The standard deviation measures an amount of variation of values of a variable about its mean. A low standard deviation indicates that the values tend to be close to the mean, also referred to as expected value, of a set of data, while a high standard deviation indicates that the values are spread out over a wider range.

For example, the determining of the principal respiratory time signal further comprises a smoothening of the principal respiratory time signal.

The smoothing may, e.g., comprise a smoothening along the time direction. For the smoothening, e.g., a Gaussian or Lanczos-kernel may be used. The smoothing may, e.g., comprise a smoothening along the time direction for short time windows, e.g., smaller 0.1 s. The smoothing for short time windows may, e.g., comprise a convolution. The smoothing may, e.g., comprise a smoothening along the time direction for longer time windows, e.g., lager 5 s. The smoothing for longer time windows may, e.g., comprise a subtraction of a convolution. Examples may have the beneficial effect, that a possible jitter and/or drift of the principal respiratory time signal may be eliminated.

For example, the determining of the principal respiratory time signal further comprises a phase-correction for the principal respiratory time signal. The phase correction comprises using the determined sensor-wise individual phase-shifts of individual respiratory time signals to determine phase-corrected individual respiratory time signals and using the phase-correction individual respiratory time signals for updating the principal respiratory time signal with a phase-corrected principal respiratory time signal to be used for the eliminating.

For example, a phase-corrected, i.e., a phase-optimized, averaged principal respiratory time signal may be computed from the individual respiratory time signals using a phase defined with respect to the principal respiratory time signal, by averaging phase-corrected individual respiratory time signals. Individual respiratory time signals with a low correlation may, e.g., not included on the averaging. The de-phased, averaged principal respiratory time signal may, e.g., be used for displaying a representative vital signal to a user on a graphical user interface (GUI).

For example, the determined principal respiratory time signal is eliminated, i.e., removed, sensor-wise from the individual datasets of the individual sensors.

For example, the determined principal respiratory time signal is eliminated in frequency-domain. For example, an FFT of the principal respiratory time signal is determined. For example, FFTs of the sensor data of the individual datasets of the individual sensors are determined. For example, the FFT of the principal respiratory time signal is subtracted sensor-wise from the FFTs of the individual datasets of the individual sensors. Before subtraction of the FFT of the principal respiratory time signal from an FFT of an individual dataset, the FFT of the principal respiratory time signal is, e.g., magnitude-peak-normalized to the FFT of the respective individual dataset. Magnitude-peak-normalized refers to an adjusting of a gain of the FFT of the principal respiratory time signal to equalize a size of largest peak of the FFT of the principal respiratory time signal to a size of a largest peak of the FFT of the respective individual dataset. Examples may have the beneficial effect of accounting for different amplitudes of the individual datasets of the individual sensors. The amplitudes may be variable for different sensors.

For example, the eliminating of the determined principal respiratory time signal comprises a phase-shifting of the principal respiratory time signal using the determined individual phase-shifts for the individual sensors. The individual phase-shifts used for phase-shifting the principal respiratory time signal against the individual datasets of the individual sensors may, e.g., be determined using an optimal cross-correlation approach. For example, a range of time phase-shifts [-T ... +T], i.e., negative and positive lags, is applied the individual dataset and a correlation with the principal respiratory time signal is determined for these different phase-shifts. A phase-shift, for which the correlation value is maximized, is selected as the individual phase-shift of the respective individual dataset.

For the eliminating, the determined principal respiratory time signal is, e.g., phase-shifted using the phase-shift determined for the individual sensor, from the individual dataset of which the principal respiratory time signal is to be eliminated. Examples may have the beneficial effect of accounting for different phase-drifts of the individual datasets of the individual sensors. Phases may differ between different sensors due to their different positions within the two-dimensional sensor array. Taking into account phase-shifts of the individual datasets of the individual sensors may, e.g., be in particular beneficial for a correct calculation of the imaginary part of the FFT of the principal respiratory time signal.

For example, the determined principal respiratory time signal is eliminated in time-domain. For the eliminating the determined principal respiratory, the determined individual phase-shifts for the individual sensors are used. The eliminating of the determined principal respiratory time signal may, e.g., comprise a phase-shifting of the principal respiratory time signal using the determined individual phase-shifts for the individual sensors. For the eliminating, the determined principal respiratory time signal is, e.g., phase-shifted using the phase-shift determined for the individual sensor, from the individual dataset of which the principal respiratory time signal is to be eliminated. Examples may have the beneficial effect of accounting for different phase-drifts of the individual datasets of the individual sensors. Phases may differ between different sensors due to their different positions within the two-dimensional sensor array.

The individual phase-shifts used for phase-shifting the principal respiratory time signal against the individual datasets of the individual sensors may, e.g., be determined using an optimal cross-correlation approach. For example, a range of time phase-shifts [-T ... +T], i.e., negative and positive lags, is applied the individual dataset and a correlation with the principal respiratory time signal is determined for these different phase-shifts. A phase-shift, for which the correlation value is maximized, is selected as the individual phase-shift of the respective individual dataset.

The principal respiratory time signal is, e.g., shifted accordingly. Furthermore, a fit of offset and scale, e.g., a linear fit, is performed between the phase-shifted principal respiratory time signal and the sensor data of the respective individual dataset. The result of the fit of the principal respiratory time signal is subtracted from the individual dataset resulting in an individual residual dataset. The residual sensor data of the resulting individual residual dataset may be assumed to be caused by the cardiac signal only.

For example, the eliminating of the determined principal respiratory time signal is carried out for each individual sensor of the two-dimensional sensor array, i.e., for each individual sensor dataset.

For example, the determining of the principal cardiac time signal further comprises a weighting of the residual sensor data of the individual residual sensor datasets within the second time window. The residual sensor data of the individual residual sensor datasets being weighted within the second time window, e.g., using a window weighting function. The window weighting function may, e.g., be a symmetric or an asymmetric function. For example, a symmetric Gaussian or Lanczos function may be used as a symmetric window weighting function. For example, an asymmetric window weighting function may be used, which gives less weight to points of time more distant from the second reference point of time compared to less distant points of time, e.g., most recent signal values, which are given more weight.

For example, the weighting is carried out for each individual sensor of the two-dimensional sensor array, i.e., for each individual residual sensor dataset.

For example, the determining of the principal cardiac time signal further comprises a filtering of the residual sensor data comprised by the set of residual sensor data. The filtering comprises a selecting of residual signal data from a predefined second frequency range of cardiac signal frequencies.

For example, confounding non-cardiac signals may be rejected by considering only signals showing a significant frequency amplitude in an appropriate frequency range, i.e., a predefined second frequency range of cardiac signal frequencies. The predefined second frequency range of cardiac signal frequencies may, e.g., be a frequency range from 0.8 Hz to 2.2 Hz.

For example, the filtering carried out for each individual sensor of the two-dimensional sensor array, i.e., for each individual residual sensor dataset.

For example, the determining of the principal cardiac time signal further comprises an averaging of the individual residual sensor data over a plurality of adjacent time windows comprised by the individual residual sensor datasets.

For example, the individual residual sensor data may be averaged over a plurality of adjacent time windows, e.g., second time windows, reaching further into the past. For the averaging, e.g., an exponential moving average (EMA) may be used, which may, e.g., give more weight to a most recent time window compared to time windows further in the past. An exponential moving average, also referred to as an exponentially weighted moving average (EWMA), is a first-order infinite impulse response filter that applies weighting factors which decrease exponentially. The weighting for each older datum decreases exponentially, without reaching zero. Examples may have the beneficial effect, that a numerical stability of the determining of the principal cardiac time signal may be increased.

For example, the averaging of the individual residual sensor data over the plurality of adjacent time windows is carried out for each individual sensor of the two-dimensional sensor array, i.e., for each individual residual sensor dataset.

For example, the determining of the principal cardiac time signal further comprises a cleansing of the individual residual sensor data comprised by the individual residual sensor datasets. The cleansing comprises subtracting of one or more contributions to the individual residual sensor data, which are negligible for the determining of the principal cardiac time signal.

The cleansing of the individual residual sensor data may, e.g., comprise a cleansing of irrelevant frequencies and/or a cleansing of a sensor drift of the individual sensor using to acquire the respective individual residual sensor data.

The cleansing may, e.g., be applied in frequency-domain. For example, a complex Fast Fourier Transform (FFT) may be applied to individual residual sensor data. A DC-component and/or non-relevant frequency ranges, e.g., above and below a pre-defined threshold, of the FFT may be down-weighted. To the resulting adjusted FFT values, e.g., an inverse FFT may be applied.

The cleansing may, e.g., be applied in frequency-domain. The cleansing may, e.g., comprise a subtraction of a weighted mean of the individual residual sensor data. The cleansing may, e.g., comprise a peak normalization, e.g., a constant amount of gain may be added to the individual residual sensor data to bring a peak of the individual residual sensor data to a pre-defined level. The cleansing may, e.g., comprise a smoothing of the individual residual sensor data. The smoothening may, e.g., comprise a convolution with a short kernel, e.g., 0.1 s, and a subtraction of a convolution with a long kernel, e.g., 5 s. The short kernel may, e.g., a binomial, a Gaussian, or a Lanczos kernel.

For example, the cleansing is carried out for each individual sensor of the two-dimensional sensor array, i.e., for each individual residual sensor dataset.

For example, the determining of the principal cardiac time signal further comprises averaging over the individual residual sensor data comprised by the individual residual sensor datasets.

For example, the averaging is executed in Fourier space by determining Fourier transforms using the individual residual sensor data and averaging the resulting Fourier transforms. For example, the averaging is executed in Fourier space by determining Fast Fourier transforms using the individual residual sensor data and averaging the resulting Fast Fourier transforms.

For example, FFTs of the individual residual datasets are determined. For example, the FFTs of the individual residual datasets, e.g., of all the individual residual datasets, are averaged into an overall FFT. The overall FFT may, e.g., be used to provide a periodogram of FFT magnitudes. For example, possible phase shifts may be expected to be small in contrast to respiratory-related phase shifts. Furthermore, the possible phase shifts may be assumed to be irrelevant for the determining of the principal cardiac time signal, because possible phase shifts may only manifest in the complex part of the FFT.

The overall periodogram may, e.g., be searched for a peak in a cardiac frequency range of interest, e.g., a frequency range from 0.8 Hz to 2.5 Hz, and a peak frequency found within the cardiac frequency range of interest may be adopted as the current heart rate.

For example, the determining of the principal cardiac time signal further comprises a refining of the principal cardiac time signal. The refining of the principal cardiac time signal comprises successively neglecting contributions of individual residual sensor datasets for the averaging, checking, whether the neglecting results in an improving of a peak-to-background-ratio, and, in response to determining an improving, removing the respective contribution from the determined principal cardiac time signal.

A determined overall FFT of the individual residual datasets may, e.g., be refined using an iterative removal of outliers and/or of non-contributing confounders. For example, each FFT of an individual residual dataset of the plurality of individual sensors may, e.g., be tentatively left out of the overall FFT-averaging for test purposes. If a peak-to-background-ratio is improving by leaving out an FFT of an individual residual dataset, then the contribution of the respective individual sensor to the overall FFT, i.e., the FFT of the individual residual dataset, is disregarded. Examples may have the beneficial effect of implementing an effecting outlier removal and/or removal of non-contributing confounders. After the refining, the refined overall FFT may, e.g., be transformed back into time domain using an inverse FFT. The result, i.e., the principal cardiac time signal may, e.g., be used to determine time points of a last and/or next expected heartbeat.

For example, the method further comprises determining a two-dimensional cardiac data representation descriptive of relations between individual cardiac time signals comprised by the individual residual datasets of the individual sensors. The output cardiac signal data further comprises the two-dimensional cardiac data representation.

For example, the two-dimensional cardiac data representation comprises a cardiac signal correlation map.

A correlation map may, e.g., be constructed from the individual cardiac time signals, i.e., the individual time series determined for the individual residual sensor of the individual sensors. The construction of the correlation map may, e.g., comprise a computing of linear cross-correlations between sensor-specific time series, i.e., individual cardiac time signals and the principal cardiac time signal. The individual respiratory time signals may, e.g., be short- and/or long-time-window-filtered, i.e., smoothened for the computing of the cross-correlations. The linear cross-correlations may, e.g., be optimized by applying a range of time phase-shifts [-T ... +T], i.e., negative and positive lags, to the individual respiratory time signals and adopting an optimum of the resulting correlations as a correlation value for the correlation map. The resulting correlation values may, e.g., be arranged into a color map using the spatial positions of the pressure sensors. For example, positive and negative correlations may be translated into different color hues, respectively, while a color intensity may, e.g., be determined by a magnitude of a local phase-optimized correlation.

For example, the two-dimensional cardiac data representation comprises a cardiac signal covariance map.

A covariance map may, e.g., be constructed from the individual cardiac time signals of the individual sensors in a manner similar to the correlation map, e.g., shift-optimized. While a linear correlation is normalized, i.e., divided by a univariance of both the principal cardiac time signal as well as the individual cardiac time signal, a covariance for this map may, e.g., be normalized only by a principal variance. Thus, an amplitude of the cardiac-related motion may be signified locally for each sensor.

For example, a correlation map and a covariance map be composited into a single map, e.g., by using different color scales, thus highlighting, e.g., hot spots of highest cardiac motion amplitude on the extended background of correlated, but possible weaker, motions.

For example, the two-dimensional cardiac data representation comprises a cardiac signal phase-shift map.

A phase-shift map may, e.g., be constructed from the individual cardiac time signals of the individual sensors in a manner similar to the correlation map and/or the covariance map by mapping a phase-shift, i.e., a positive or negative time lag for which an optimal cross-correlation is achieved.

Exemplary maps of cardiac signal data may, e.g., yield a comprehensive visualization of cardiac related motion patterns, thus allowing for an improved imaging planning and/or controlling for acquiring medical imaging data of the subject lying on the two-dimensional sensor array.

For example, the method further comprises outputting the principal respiratory time signal.

For example, the method further comprises determining a two-dimensional respiratory data representation descriptive of relations between individual respiratory time signals comprised by the individual datasets of the individual sensors and outputting the two-dimensional respiratory data representation.

For example, the two-dimensional respiratory data representation comprises a respiratory signal correlation map.

A correlation map may, e.g., be constructed from the individual respiratory time signals, i.e., the individual time series acquired by the individual sensors. The construction of the correlation map may, e.g., comprise a computing of linear cross-correlations between sensor-specific time series, i.e., individual respiratory time signals and the principal respiratory time signal. The individual respiratory time signals may, e.g., be short- and/or long-time-window-filtered, i.e., smoothened for the computing of the cross-correlations. The linear cross-correlations may, e.g., be optimized by applying a range of time phase-shifts [-T ... +T], i.e., negative and positive lags, to the individual respiratory time signals and adopting an optimum of the resulting correlations as a correlation value for the correlation map. This optimization may, e.g., account for non-synchronous motions of different body parts. The resulting correlation values may, e.g., be arranged into a color map using the spatial positions of the pressure sensors. For example, positive and negative correlations may be translated into different color hues, respectively, while a color intensity may, e.g., be determined by a magnitude of a local phase-optimized correlation.

The correlation map may, e.g., be beneficial to visualize a positioning of breathing related body parts of the subject on the two-dimensional sensor array and/or to visualize which body parts are moving co-variant and which body parts move contra-variant with the principal respiratory motion.

For example, the two-dimensional respiratory data representation comprises a respiratory signal covariance map.

A covariance map may, e.g., be constructed from the individual respiratory time signals, i.e., the individual time series acquired by the individual sensors in a manner similar to the correlation map, e.g., shift-optimized. While a linear correlation is normalized, i.e., divided by a univariance of both the principal respiratory time signal as well as the individual respiratory time signal, a covariance for this map may, e.g., be normalized only by a principal variance. Thus, an amplitude of the breathing-related motion may be signified locally for each sensor.

The covariance map may, e.g., be beneficial to visualize, where a most significant respiratory-related motion takes place.

For example, a correlation map and a covariance map be composited into a single map, e.g., by using different color scales, thus highlighting hot spots of highest breathing motion amplitude on the extended background of correlated, but possible weaker, motions.

For example, the two-dimensional respiratory data representation comprises a respiratory signal phase-shift map.

A phase-shift map may, e.g., be constructed from the individual respiratory time signals, i.e., the individual time series acquired by the individual sensors in a manner similar to the correlation map and/or the covariance map by mapping a phase-shift, i.e., a positive or negative time lag for which an optimal cross-correlation is achieved.

The phase-shift map may, e.g., be beneficial to visualize which body parts of a subject are moving pre- or post-temporal with the principal respiratory motion.

The phase-shift map may, e.g., be used for medical imaging planning, such that a certain body part of the subject is scanned, i.e., imaging data thereof being acquired, at a time point of least motion to avoid image motion artifacts.

The phase-shift map may, e.g., be used to trigger and/or gate an imaging data acquisition and/or reconstruction by either selecting the sensors to be used depending on their location with respect to a target anatomy to be imaged or by applying a corresponding trigger delay, where the trigger points may, e.g., be detected on the de-phased, averaged principal respiratory time signal.

A spatial pattern and/or extracted phase shifts of the respiratory signal may, e.g., be used to compute body-part optimized trigger points for an imaging data acquisition and reconstruction processes.

Exemplary maps of respiratory signal data may, e.g., yield a comprehensive visualization of respiratory related motion patterns, thus allowing for an improved imaging planning and/or controlling for acquiring medical imaging data of the subject lying on the two-dimensional sensor array. Furthermore, a stratifying of breathing patterns of the subjects into subtypes may, e.g., be enabled, such as abdominal vs chest breathing, and, e.g., be used for breathing guidance purposes.

Examples may have the beneficial effect of providing two-dimensional distribution of the respiratory signal data. Two-dimensional distribution of the respiratory signal data may be beneficial compared to, e.g., a single-point respiratory signal, which may, e.g., be acquired using a belt or air bellow and may not be sufficiently descriptive to reflect various breathing-related body motions required for motion-blur-free imaging.

For example, the method further comprises determining a two-dimensional respiratory-cardiac data representation descriptive of relations between individual respiratory time signals comprised by the individual datasets of the individual sensors and the individual cardiac time signals comprised by the individual residual datasets of the individual sensors as well as outputting the two-dimensional respiratory-cardiac data representation.

Exemplary maps of respiratory and cardiac signal data may, e.g., yield a comprehensive visualization of respiratory-cardiac related motion patterns, thus allowing for an improved imaging planning and/or controlling for acquiring medical imaging data of the subject lying on the two-dimensional sensor array. Furthermore, a stratifying of breathing patterns of the subjects into subtypes may, e.g., be enabled, such as abdominal vs chest breathing, and, e.g., be used for breathing guidance purposes.

In an example, a computer program for extracting cardiac signal data from a set of sensor data acquired using a plurality of pressure sensors of a two-dimensional sensor array provided. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array. The computer program comprises machine executable instructions for execution by a computational system. Execution of the machine executable instructions causes the computational system to perform the method of any of the examples disclosed herein.

For example, the execution of the machine executable instructions causes the computational system to receive the set of sensor data. A principal respiratory time signal of a respiratory motion pattern for the subject is determined using the set of sensor data. The determined principal respiratory time signal is a common one-dimensional respiratory time signal for the plurality of sensors. Sensor-wise individual phase-shifts of individual respiratory time signals comprised by the individual datasets of the individual sensors are determined. The determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. A principal cardiac time signal of a cardiac motion pattern for the subject is determined using the set of residual sensor data. The determined principal cardiac time signal is a common one-dimensional cardiac time signal for the plurality of sensors. Cardiac signal data comprising the principal cardiac time signal is output.

For example, a computer program product for extracting cardiac signal data from a set of sensor data acquired using a plurality of pressure sensors of a two-dimensional sensor array may be provided. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array. The computer program product may comprise a non-transitory computer readable storage medium having program instructions embodied therewith for execution by a computational system. Execution of the machine executable instructions causes the computational system to perform the method of any of the examples disclosed herein.

In an example, a medical system comprises a computational system for extracting cardiac signal data from a set of sensor data acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

The computational system comprises a memory storing machine executable instructions. Execution of the machine executable instructions causes the computational system to perform the method of any of the examples disclosed herein.

For example, the execution of the machine executable instructions causes the computational system to receive the set of sensor data. A principal respiratory time signal of a respiratory motion pattern for the subject is determined using the set of sensor data. The determined principal respiratory time signal is a common one-dimensional respiratory time signal for the plurality of sensors. Sensor-wise individual phase-shifts of individual respiratory time signals comprised by the individual datasets of the individual sensors are determined. The determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. A principal cardiac time signal of a cardiac motion pattern for the subject is determined using the set of residual sensor data. The determined principal cardiac time signal is a common one-dimensional cardiac time signal for the plurality of sensors. Cardiac signal data comprising the principal cardiac time signal is output.

For example, the medical system further comprises the two-dimensional sensor array.

For example, the medical system further comprises a medical imaging system. The machine executable instructions further are configured for controlling the medical imaging system. The execution of the machine executable instructions further causes the computational system to use the output cardiac signal data for controlling an imaging data acquisition using the medical imaging system. For example, the output cardiac signal data may be used for triggering an imaging data acquisition using the medical imaging system. The medical imaging system may, e.g., be an MRI system or a CT system.

Examples may have the beneficial effect that, e.g., an automated imaging data acquisition using the medical imaging system may be enabled.

The execution of the machine executable instructions may, e.g., further causes the computational system to output respiratory signal data. The execution of the machine executable instructions may, e.g., further causes the computational system to use the output respiratory signal data for controlling an imaging data acquisition using the medical imaging system. For example, the output respiratory signal data may be used for triggering an imaging data acquisition using the medical imaging system.

The execution of the machine executable instructions may, e.g., further causes the computational system to use the output cardiac signal data and the output respiratory signal data for controlling an imaging data acquisition using the medical imaging system. For example, the output cardiac signal data and the output respiratory signal data may be used for triggering an imaging data acquisition using the medical imaging system.

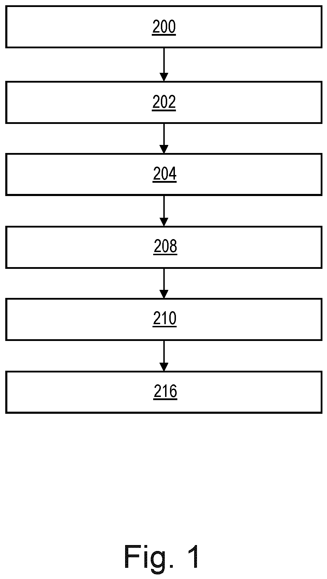

Fig. 1 shows an exemplary method for extracting cardiac signal data from a set of sensor data acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

In block 200, the set of sensor data is received. In block 202, a principal respiratory time signal of a respiratory motion pattern for the subject is determined using the set of sensor data. The determined principal respiratory time signal is a common one-dimensional respiratory time signal for the plurality of sensors. For example, the determined principal respiratory time signal is smoothened. The principal respiratory time signal may, e.g., be smoothened using a convolution withing shorter time windows and/or using a subtraction of a convolution within longer time windows. In block 204, sensor-wise individual phase-shifts of individual respiratory time signals comprised by the individual datasets of the individual sensors are determined. In block 208, the determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. In block 210, a principal cardiac time signal of a cardiac motion pattern for the subject is determined using the set of residual sensor data. The determined principal cardiac time signal is a common one-dimensional cardiac time signal for the plurality of sensors. In block 216, cardiac signal data comprising the principal cardiac time signal is output.

The output cardiac signal data may, e.g., be used for monitoring the subject. The output cardiac signal data may, e.g., be used for planning and/or controlling a medical imaging data acquisition using a medical imaging system. The output cardiac signal data may, e.g., be used for triggering a medical imaging data acquisition executed using a medical imaging system. The output cardiac signal data may, e.g., be used for reconstructing medical images using medical imaging data acquired with a medical imaging system.

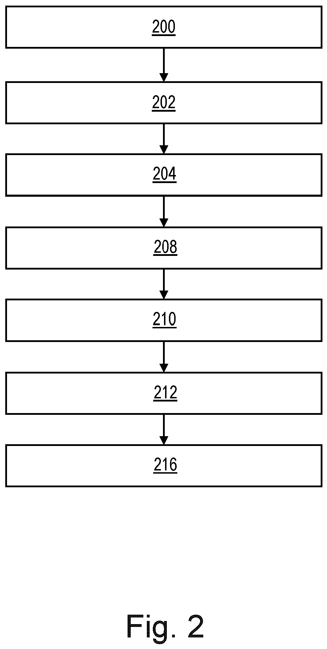

Fig. 2 shows another exemplary method for extracting cardiac signal data from a set of sensor data acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

In block 200, the set of sensor data is received. In block 202, a principal respiratory time signal of a respiratory motion pattern for the subject is determined using the set of sensor data. The determined principal respiratory time signal is a common one-dimensional respiratory time signal for the plurality of sensors. For example, the determined principal respiratory time signal is smoothened. The principal respiratory time signal may, e.g., be smoothened using a convolution withing shorter time windows and/or using a subtraction of a convolution within longer time windows. In block 204, sensor-wise individual phase-shifts of individual respiratory time signals comprised by the individual datasets of the individual sensors are determined. In block 208, the determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. In block 210, a principal cardiac time signal of a cardiac motion pattern for the subject is determined using the set of residual sensor data. The determined principal cardiac time signal is a common one-dimensional cardiac time signal for the plurality of sensors. In block 212, a two-dimensional cardiac data representation descriptive of relations between individual cardiac time signals comprised by the individual residual datasets of the individual sensors is determined. The two-dimensional cardiac data representation, e.g., comprises a cardiac signal correlation map. The two-dimensional cardiac data representation, e.g., comprises a cardiac signal covariance map. The two-dimensional cardiac data representation, e.g., comprises a cardiac signal phase-shift map. In block 216, cardiac signal data comprising the principal cardiac time signal is output. The output cardiac signal data further comprises the two-dimensional cardiac data representation.

The output cardiac signal data may, e.g., be used for monitoring the subject. The output cardiac signal data may, e.g., be used for planning and/or controlling a medical imaging data acquisition using a medical imaging system. The output cardiac signal data may, e.g., be used for triggering a medical imaging data acquisition executed using a medical imaging system. The output cardiac signal data may, e.g., be used for reconstructing medical images using medical imaging data acquired with a medical imaging system.

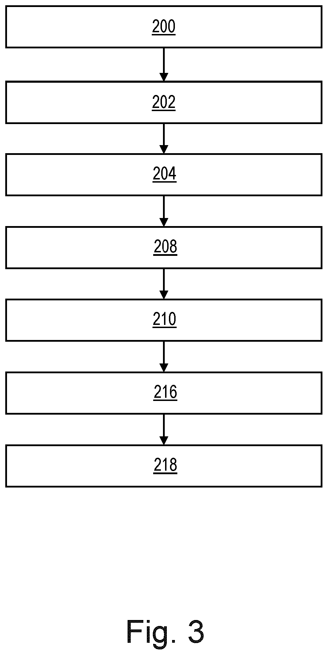

Fig. 3 shows another exemplary method for extracting cardiac and respiratory signal data from a set of sensor data acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

In block 200, the set of sensor data is received. In block 202, a principal respiratory time signal of a respiratory motion pattern for the subject is determined using the set of sensor data. The determined principal respiratory time signal is a common one-dimensional respiratory time signal for the plurality of sensors. For example, the determined principal respiratory time signal is smoothened. The principal respiratory time signal may, e.g., be smoothened using a convolution withing shorter time windows and/or using a subtraction of a convolution within longer time windows. In block 204, sensor-wise individual phase-shifts of individual respiratory time signals comprised by the individual datasets of the individual sensors are determined. In block 208, the determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. In block 210, a principal cardiac time signal of a cardiac motion pattern for the subject is determined using the set of residual sensor data. The determined principal cardiac time signal is a common one-dimensional cardiac time signal for the plurality of sensors. In block 216, cardiac signal data comprising the principal cardiac time signal is output. In block 218, respiratory signal data comprising the principal respiratory time signal is output.

The output cardiac and/or respiratory signal data may, e.g., be used for monitoring the subject. The output cardiac and/or respiratory signal data may, e.g., be used for planning and/or controlling a medical imaging data acquisition using a medical imaging system. The output cardiac and/or respiratory signal data may, e.g., be used for triggering a medical imaging data acquisition executed using a medical imaging system. The output cardiac and/or respiratory sensor data may, e.g., be used for reconstructing medical images using medical imaging data acquired with a medical imaging system.

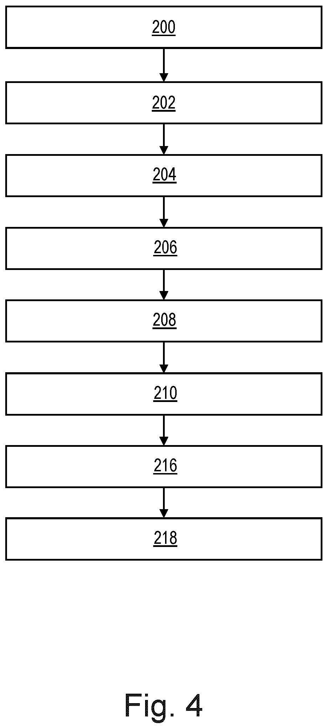

Fig. 4 shows another exemplary method for extracting cardiac and respiratory signal data from a set of sensor data acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

In block 200, the set of sensor data is received. In block 202, a principal respiratory time signal of a respiratory motion pattern for the subject is determined using the set of sensor data. The determined principal respiratory time signal is a common one-dimensional respiratory time signal for the plurality of sensors. For example, the determined principal respiratory time signal is smoothened. The principal respiratory time signal may, e.g., be smoothened using a convolution withing shorter time windows and/or using a subtraction of a convolution within longer time windows. In block 204, sensor-wise individual phase-shifts of individual respiratory time signals comprised by the individual datasets of the individual sensors are determined. In block 206, a two-dimensional respiratory data representation descriptive of relations between individual respiratory time signals comprised by the individual datasets of the individual sensors is determined. The two-dimensional respiratory data representation, e.g., comprises a respiratory signal correlation map. The two-dimensional respiratory data representation, e.g., comprises a respiratory signal covariance map. The two-dimensional respiratory data representation, e.g., comprises a respiratory signal phase-shift map. In block 208, the determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. In block 210, a principal cardiac time signal of a cardiac motion pattern for the subject is determined using the set of residual sensor data. The determined principal cardiac time signal is a common one-dimensional cardiac time signal for the plurality of sensors. In block 216, cardiac signal data comprising the principal cardiac time signal is output. In block 218, respiratory signal data comprising the two-dimensional respiratory data representation is output. For example, the signal data may in addition comprise the principal respiratory time signal.

The output cardiac and/or respiratory signal data may, e.g., be used for monitoring the subject. The output cardiac and/or respiratory signal data may, e.g., be used for planning and/or controlling a medical imaging data acquisition using a medical imaging system. The output cardiac and/or respiratory signal data may, e.g., be used for triggering a medical imaging data acquisition executed using a medical imaging system. The output cardiac and/or respiratory sensor data may, e.g., be used for reconstructing medical images using medical imaging data acquired with a medical imaging system.

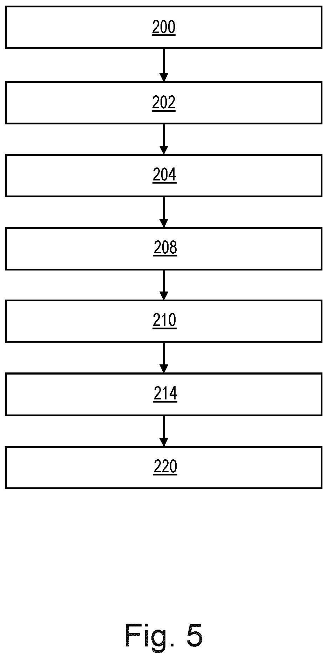

Fig. 5 shows another exemplary method for extracting cardiac signal data from a set of sensor data acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

In block 200, the set of sensor data is received. In block 202, a principal respiratory time signal of a respiratory motion pattern for the subject is determined using the set of sensor data. The determined principal respiratory time signal is a common one-dimensional respiratory time signal for the plurality of sensors. For example, the determined principal respiratory time signal is smoothened. The principal respiratory time signal may, e.g., be smoothened using a convolution withing shorter time windows and/or using a subtraction of a convolution within longer time windows. In block 204, sensor-wise individual phase-shifts of individual respiratory time signals comprised by the individual datasets of the individual sensors are determined. In block 208, the determined principal respiratory time signal is eliminated sensor-wise from the individual datasets of the individual sensors using the determined individual phase-shifts for the individual sensors. The eliminating results in a set of residual sensor data comprising a plurality of individual residual sensor datasets with residual sensor data for the individual sensors. In block 210, a principal cardiac time signal of a cardiac motion pattern for the subject is determined using the set of residual sensor data. The determined principal cardiac time signal is a common one-dimensional cardiac time signal for the plurality of sensors. In block 214, a two-dimensional respiratory-cardiac data representation descriptive of relations between individual respiratory time signals comprised by the individual datasets of the individual sensors and the individual cardiac time signals comprised by the individual residual datasets of the individual sensors is determined. In block 220, cardiac and respiratory signal data comprising the principal cardiac time signal is output. The cardiac and respiratory signal data further comprises the two-dimensional respiratory-cardiac data representation. For example, the cardiac and respiratory signal data may in addition comprise the principal respiratory time signal.

The output cardiac and respiratory signal data may, e.g., be used for monitoring the subject. The output cardiac and respiratory signal data may, e.g., be used for planning and controlling a medical imaging data acquisition using a medical imaging system. The output cardiac and respiratory signal data may, e.g., be used for triggering a medical imaging data acquisition executed using a medical imaging system. The output cardiac and respiratory sensor data may, e.g., be used for reconstructing medical images using medical imaging data acquired with a medical imaging system.

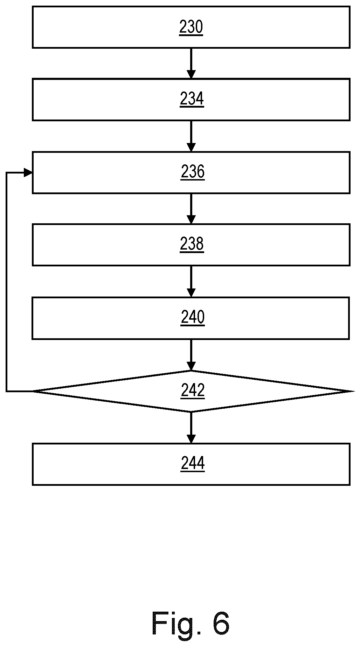

Fig. 6 shows an exemplary method for determining a principal respiratory time signal of a respiratory motion pattern for the subject using a set of sensor data. The set of sensor data is acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array.

In block 230, the sensor data comprised by the set of sensor data is, e.g., filtered. The filtering comprises a selecting of signal data from a predefined first frequency range of respiratory signal frequencies. For example, a successive averaging of selected individual respiratory time signals comprised by the individual datasets of the individual sensors is executed using a covariance analysis for the selecting. In block 234, as a starting step of the successive averaging, a pair of individual respiratory time signals is averaged, for which a highest covariance compared to all other pairs of individual respiratory time signals is determined. In block 236, covariances of a current averaged respiratory time signal and remaining individual respiratory time signals are determined. For a first execution of block 236, e.g., the averaged pair of individual respiratory time signals resulting from block 234 is used as the current averaged respiratory time signal. For further iterative executions of block 236 the results of block 240 are used as updates for the current averaged respiratory time signal being used in block 236. In block 238, a remaining individual respiratory time signal is selected, for which a highest covariance with respect to the current averaged respiratory time signal is determined compared to all other remaining individual respiratory time signals. In block 240, the current averaged respiratory time signal and the selected remaining individual respiratory time signal are averaged. In block 242, it is checked, whether a termination criterion is satisfied. In case the termination criterion is satisfied, the successive averaging of selected individual respiratory time signals is continued with block 236 using the result of block 240 as an updated current averaged respiratory time signal. In case the termination criterion is satisfied, the successive averaging of selected individual respiratory time signals is terminated, and the method continues with block 244. In block 244, e.g., a phase-correction for the principal respiratory time signal is executed. The phase-correction comprises determining phase-corrected individual respiratory time signals using the determined sensor-wise individual phase-shifts of individual respiratory time signals. The phase-correction individual respiratory time signals are used for updating the principal respiratory time signal with a phase-corrected principal respiratory time signal. The updated principal respiratory time signal is determined to be used for the eliminating of the determined principal respiratory time signal sensor-wise from the individual datasets of the individual sensors using the determined individual phase-shifts for the individual sensors.

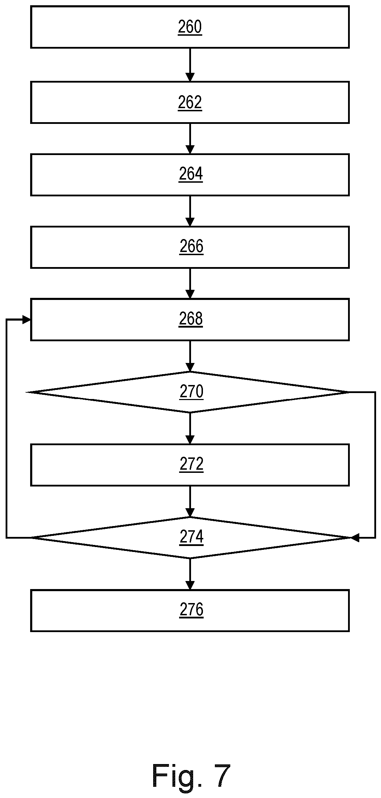

Fig. 7 shows an exemplary method for determining a principal cardiac time signal of a cardiac motion pattern for the subject using a set of residual sensor data. A set of sensor data is acquired using a plurality of pressure sensors of a two-dimensional sensor array. The set of sensor data comprises a plurality of individual sensor datasets with sensor data acquired by the individual sensors of the sensor array. The sensor data of the individual sensor datasets is descriptive of pressure changes detected over time by the individual sensors due to motions of a subject lying on the sensor array. The set of residual sensor data results from eliminating a determined principal respiratory time signal sensor-wise from the individual datasets of the individual sensors using determined individual phase-shifts for the individual sensors. The set of residual sensor data comprises a plurality of individual residual sensor datasets with residual sensor data for the individual sensors.

In block 260, the residual sensor data comprised by the set of residual sensor data is, e.g., filtered. The filtering comprises a selecting of residual signal data from a predefined second frequency range of cardiac signal frequencies. In block 262, the individual residual sensor data is e.g., averaged over a plurality of adjacent time windows comprised by the individual residual sensor datasets. In block 264, the individual residual sensor data comprised by the individual residual sensor datasets is, e.g., cleansed. The cleansing comprises subtracting of one or more contributions to the individual residual sensor data, which are negligible for the determining of the principal cardiac time signal. In block 266, it is averaged over the individual residual sensor data comprised by the individual residual sensor datasets. The averaging, e.g., is executed in Fourier space by determining Fourier transforms, e.g., Fast Fourier transforms, using the individual residual sensor data and averaging the resulting Fourier transforms, e.g., Fast Fourier transforms. The determining of the principal cardiac time signal, e.g., further comprises a refining of the principal cardiac time signal. In block 268, a contribution of an individual residual sensor dataset for the averaging over the individual residual sensor data is neglected. In block 270, it is checked, whether the neglecting results in an improving of a peak-to-background-ratio. In response to determining an improving of the peak-to-background-ratio in block 270, the method continuous with block 272. In block 272, the respective contribution from the determined principal cardiac time signal is removed. In in response to determining, that the peak-to-background-ratio in block 270 is not improved, the method continuous with block 274. In block 274, it is checked, whether the contribution neglected in block 268 was the last one of the contributions of individual residual sensor datasets. In case the contribution neglected in block 268 was not the last one of the contributions of individual residual sensor datasets, the method continuous with block 268 and a neglecting of a next one of the contributions of individual residual sensor datasets. In case the contribution neglected in block 268 was not the last one of the contributions of individual residual sensor datasets, the method continuous with block 276. In block 276, the refining of the principal cardiac time signal is ended.

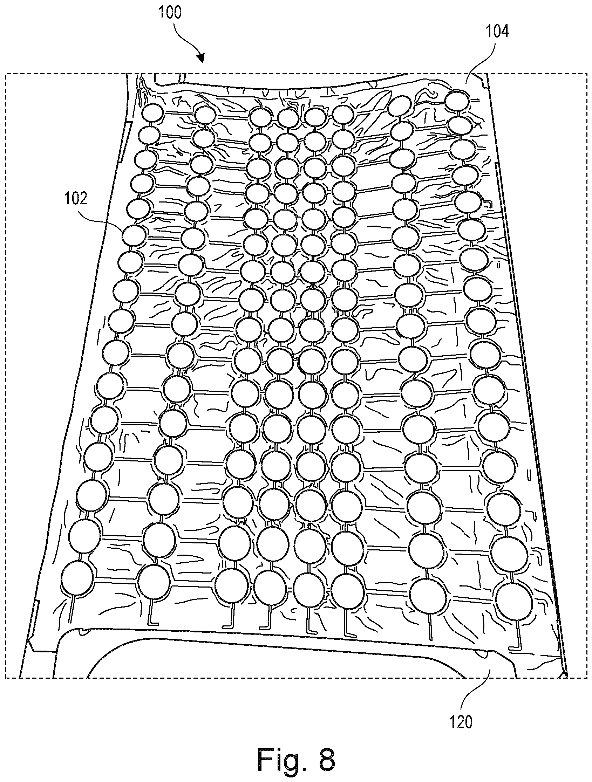

Fig. 8 shows an exemplary two-dimensional sensor array 100 comprising a plurality of pressure sensors 102. The two-dimensional sensor array 100 may, e.g., be embedded into a mattress 104. The respective mattress 104 may, e.g., be provided in form of a mattress-like thin blanket comprising the two-dimensional sensor array 100. The mattress 104 may, e.g., be configured to be placed underneath a subject, such that the respective subject may, e.g., lay down on to the mattress 104 with the two-dimensional sensor array 100. For this purpose, mattress 104 may, e.g., be arranged on a subject support 120 of a medical imaging system, such as, e.g., an MRI or CT system. Mattress 104 may, e.g., be a single-use item.

The pressure sensors 102 of the two-dimensional sensor array 100 may, e.g., be provided in form of fiber-optical pressure sensors with fiber crossings, fiber-optical pressure sensors with Bragg-gratings, piezo-electrical pressure sensors, capacitive pressure sensors, and/or gas/air pocket/bellow pressure sensors.

Using fiber-optical pressure sensors may, e.g., have the beneficial effect, that they may be suitable for usage inside a gantry of a medical imaging system, such as a CT imaging system and/or an MR imaging system, without causing imaging artefacts. Fiber-optical pressure sensors may, e.g., comprise no metal parts and/or electrical wiring, thereby avoiding causing imaging artefacts, e.g., in CT imaging and/or an MRI. Especially, MRI and/or CT types of medical imaging may suffer from limitation regarding the type of pressure sensors, which might be suitable for usage such medical imaging methods. Fiber-optical pressure sensors may, e.g., be immune and non-disturbing with respect to strong magnetic and/or radio frequency fields used for acquiring MRI data.

A two-dimensional sensor array 100 with fiber-optical pressure sensors 102 may, e.g., comprise a plurality of row optical fibers and a plurality of column optical fibers. The optical fibers may, e.g., comprise polymer or plastic optical fibers (POF). The row optical fibers and the column optical fibers may, e.g., be deformable and arranged in a planar two-dimensional matrix or grid structure, e.g., a rectangular grid structure, defining a pressure sensor 102 at each crosspoint of the optical fibers. Each crosspoint defining a pressure sensor 102 may, e.g., comprise a row optical fiber in contact with a column optical fiber at an intersection of the same. Each crosspoint may, e.g., further comprise a light coupling structure configured to enhance coupling, when pressure is applied to the crosspoint, i.e., pressure sensor 102. The light coupling structure may, e.g., comprise at least one layer of mechanical light scattering material disposed in contact with at least one of the row or column optical fiber. For example, the row optical fibers and the column optical fibers may comprise fiber cores surrounded by a fiber cladding. The fiber claddings of both the row optical fibers and the column optical fibers may, e.g., be arranged for light transmissive contacts at the crosspoints. The claddings may, e.g., be sufficiently thin that a non-negligible amount of light may be coupled from the core of one fiber to the crossing fiber. A two-dimensional sensor array 100 comprising fiber-optical pressure sensors 102, e.g., as described above, may be configured to sense pressure, when light is provided to the row optical fibers, by measuring the light coupled at each crosspoint, i.e., pressure sensors 102, to its column optical fiber or vice versa. The amount of coupled light may, e.g., dependent on the pressure applied to the crosspoint, which thereby acts as a pressure sensor 102. Moreover, as only a small amount of light is coupled out of the transmitting optical fiber, when pressure is exerted on the respective crosspoint, the light power which remains in the transmitting optical fiber and which arrives at the next crosspoint may, e.g., hardly be reduced. Thus, the sensitivity of a crosspoint may, e.g., hardly be affected by pressure exerted on other crosspoints. Thus, e.g., a two-dimensional sensor array 100 with fiber-optical pressure sensors 102 having high sensitivity may be provided. With such a sensor array 100, a two-dimensional spatial distribution of pressure of a subject arranged, e.g., lying on the two-dimensional sensor array 100 may be effectively measured. Furthermore, such a two-dimensional sensor array 100 with fiber-optical pressure sensors 102 may, e.g., be easily embedded into a mattress 104.

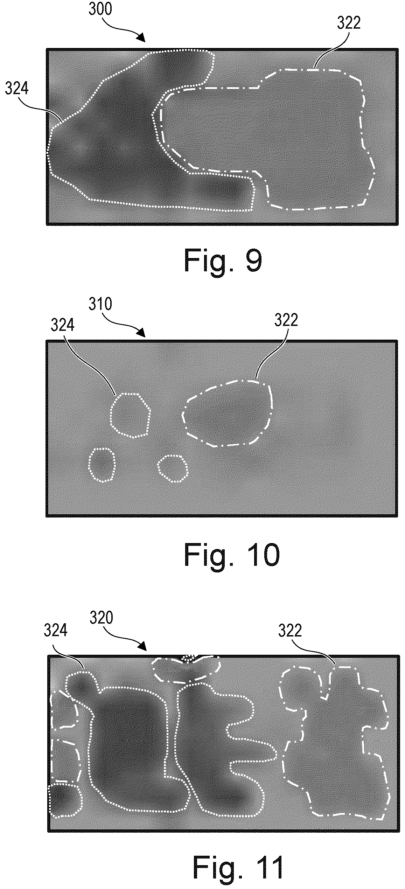

Fig. 9 shows an exemplary correlation map 300 for respiratory time signals acquired by individual pressure sensors of an exemplary two-dimensional sensor array. Correlation map 300 shows correlation values for correlations of the individual respiratory time signals acquired by the individual pressure sensors and the determined principal respiratory time signal. For generating the correlation map 300, e.g., linear cross-correlations between the individual respiratory time signals and the principal respiratory time signal are computed. The individual respiratory time signals may, e.g., be short- and/or long-time-window-filtered, i.e., smoothened for the computing of the cross-correlations. The linear cross-correlations for the individual respiratory time signals may, e.g., be optimized by applying a range of time phase-shifts [-T ... +T], i.e., negative and positive lags, to the individual respiratory time signals and adopting an optimum of the resulting correlations as a correlation value for the correlation map. This optimization may, e.g., account for non-synchronous motions of different body parts. The resulting correlation values may, e.g., be arranged into a color map using the spatial positions of the pressure sensors. For example, positive and negative correlations may be translated into different color hues, respectively, while a color intensity may, e.g., be determined by a magnitude of a local phase-optimized correlation. Thus, sections 302 (dotted line) with a motion pattern in positive correlation with the determined principal respiratory time signal and sections 304 (dotted-dashed line) in negative correlation with the determined principal respiratory time signal may be determined.

Fig. 10 shows an exemplary covariance map 310 for respiratory time signals acquired by individual pressure sensors of an exemplary two-dimensional sensor array. The covariance map 310 shows covariance values for the individual respiratory time signals acquired by the individual pressure sensors in view of the determined principal respiratory time signal. The covariance map 310 may, e.g., be constructed from the individual time series acquired by the individual sensors in a manner similar to the correlation map 300, e.g., using a shift-optimization. While a linear correlation is normalized, i.e., divided by a univariance of both the principal respiratory time signal as well as the individual respiratory time signal, a covariance for this map 310 may, e.g., be normalized only by a principal variance. Thus, an amplitude of the breathing-related motion may be signified locally for each sensor. The resulting covariance values may, e.g., be arranged into a color map using the spatial positions of the pressure sensors. For example, positive and negative covariances may be translated into different color hues, respectively, while a color intensity may, e.g., be determined by a magnitude of a local phase-optimized covariance. Thus, sections 312 (dotted line) with a motion pattern in positive covariance with the determined principal respiratory time signal and sections 314 (dotted-dashed line) in negative covariance with the determined principal respiratory time signal may be determined. The covariance map 310 may, e.g., be beneficial to visualize, where a most significant respiratory-related motion takes place.

Fig. 11 shows an exemplary phase-shift map 320. A phase-shift map 320 as shown in Fig. 11 may, e.g., be generated individual respiratory time signals acquired by the individual sensors in a manner similar to the correlation map 300 and/or the covariance map 310 by mapping a phase-shift, i.e., a positive or negative time lag, for which an optimal cross-correlation is achieved. The resulting phase-shifts may, e.g., be arranged into a color map using the spatial positions of the pressure sensors. For example, positive and negative phase-shifts may be translated into different color hues, respectively, while a color intensity may, e.g., be determined by a magnitude of a local phase-shift. Thus, sections 322 (dotted line) with a positive phase-shift relative to the determined principal respiratory time signal and sections 324 (dotted-dashed line) with a negative phase-shift relative to the determined principal respiratory time signal may be determined. The phase-shift map 320 may, e.g., be beneficial to visualize which body parts of a subject are moving pre- or post-temporal with the principal respiratory motion. The phase-shift map 320 may, e.g., be used for medical imaging planning, such that a certain body part of the subject is scanned, i.e., imaging data thereof being acquired, at a time point of least motion to avoid image motion artifacts. The phase-shift map 320 may, e.g., further be used to trigger and/or gate an imaging data acquisition and/or reconstruction by either selecting the sensors to be used depending on their location with respect to a target anatomy to be imaged or by applying a corresponding trigger delay, where the trigger points may, e.g., be detected on the de-phased, averaged principal respiratory time signal.

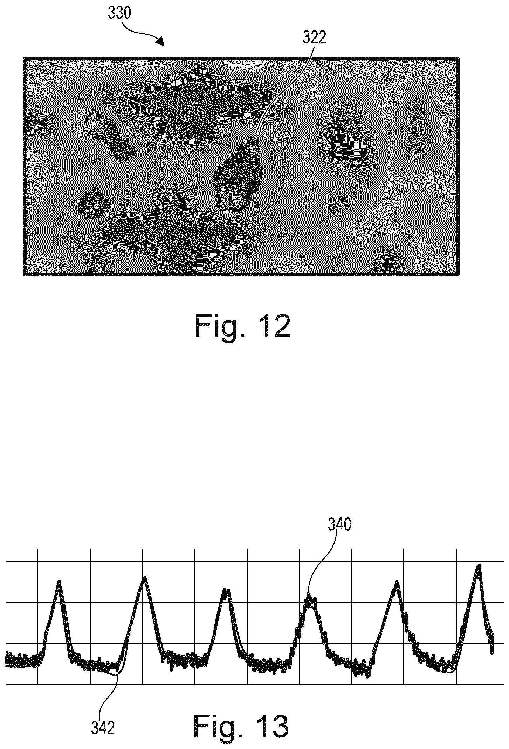

Fig. 12 shows an exemplary map 330, which combines correlation and covariance information. Map 330 is a single map, in which a correlation map, e.g., like correlation map 300, and a covariance map, e.g., like covariance map 310, have been composed into. For this purpose, e.g., different color scales may be used. Thus, e.g., hot spots 332 of highest breathing motion amplitude on the extended background of correlated, but possible weaker, motions may be highlighted.

Fig. 13 shows an exemplary principal respiratory time signal 340 of a respiratory motion pattern. The principal respiratory time signal 340 is, e.g., determined using a set of sensor data acquired by a plurality of pressure sensors of a two-dimensional sensor array. The set of sensor data comprises a plurality of individual sensor datasets with sensor data acquired by the individual sensors of the sensor array, which are descriptive of pressure changes detected over time by the individual sensors due to motions of a subject lying on the sensor array. The determined principal respiratory time signal 340 is a common one-dimensional respiratory time signal for the plurality of sensors. It may, e.g., be generated using an averaging over the individual respiratory time signals comprised by the individual datasets of the individual sensors. The determining of the principal respiratory time signal 340 may, e.g., further comprise a smoothening of the principal respiratory time signal 340 resulting in a smoothened principal respiratory time signal 342. The smoothened principal respiratory time signal 342 may, e.g., be used as the principal respiratory time signal for a further processing of the set of sensor data. It may, e.g., be used for determining sensor-wise individual phase-shifts of individual respiratory time signals comprised by the individual datasets of the individual sensors. It may, e.g., be eliminated sensor-wise from the individual datasets of the individual sensors using the determined individual phase-shifts for the individual sensors.

The smoothing may, e.g., comprise a smoothening along the time direction. For the smoothening, e.g., a Gaussian or Lanczos-kernel may be used. The smoothing may, e.g., comprise a smoothening along the time direction for short time windows, e.g., smaller 0.1 s. The smoothing for short time windows may, e.g., comprise a convolution. The smoothing may further, e.g., comprise a smoothening along the time direction for longer time windows, e.g., lager 5 s. The smoothing for longer time windows may, e.g., comprise a subtraction of a convolution. Examples may have the beneficial effect, that a possible jitter and/or drift of the principal respiratory time signal 302 may be eliminated.

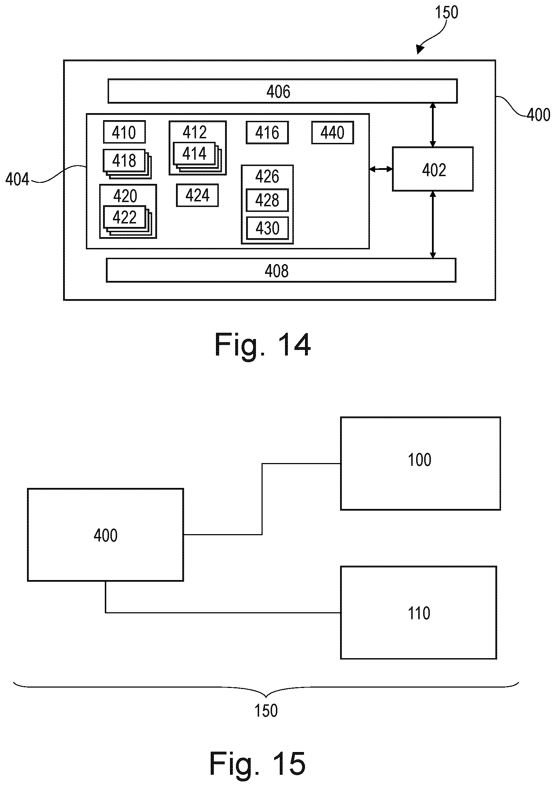

Fig. 14 shows an exemplary computational system 400 of an exemplary medical system 150. The computational system 400 may, e.g., be configured for extracting cardiac signal data from a set of sensor data acquired using a plurality of pressure sensors of a two-dimensional sensor array. The set of sensor data 412 comprises a plurality of individual sensor datasets 414 with sensor data acquired by the individual sensors of the sensor array.

The computational system 400 is shown as comprising a computational component 402. The computational component 402 is intended to represent one or more processors or processing cores or other computational elements. The computational component 402 is shown as being connected to a hardware interface 406 and a memory 404. The hardware interface 406 may enable the computational component 402 to exchange commands and data with other components, e.g., a sensor array comprising a plurality of pressure sensors and/or a medical imaging system, like a CT system or am MRI system. The hardware interface 406 may, e.g., enable the computational component 402 to control the sensor array to acquire a set of sensor data 412 with sensor data descriptive of pressure changes detected over time by the individual sensors of the sensor array due to motions of a subject lying on the sensor array. The hardware interface 406 may further, e.g., enable the computational component 402 to control a medical imaging system, like a CT system or am MRI system.

The computational system 400 is further shown as being connected to a user interface 408 which may for example enable an operator to control and operate the computational system 400 and via the computational system 400, e.g., a sensor array comprising a plurality of pressure sensors and/or a medical imaging system. The user interface 408 may, e.g., comprise an output and/or input device enabling the operator to interact with the computational system 400. The output device may, e.g., comprise a display device configured for displaying cardiac signal data 428 and/or respiratory signal data 430. Furthermore, the display device may, e.g., be configured for displaying medical images generated using a medical imaging system. The input device may, e.g., comprise a keyboard and/or a mouse enabling the operator to insert control commands for controlling the computational system 400 and via the computational system 400, e.g., a sensor array and/or a medical imaging system.

The memory 404 is shown as containing machine-executable instructions 410. The machine-executable instructions 410 enable the computational component 402 to perform controlling tasks, such as controlling a sensor array and/or a medical imaging system, to perform numerical tasks, as well as performing various signal data processing tasks, like extracting cardiac signal data 428 and/or respiratory data 430 using the set of sensor data acquired with a pressure sensor array and/or reconstructing images using imaging data acquired with a medical imaging system. The machine-executable instructions 410 may, e.g., enable the computational component 402 and thus the computational system 400 to execute any of the methods of Fig. 1 to 7.

The memory 404 is further shown as containing a set of sensor data 412 acquired using a plurality of pressure sensors of a two-dimensional sensor array. The set of sensor data 412 comprises a plurality of individual sensor datasets 414 with sensor data acquired by the individual sensors of the sensor array. The sensor data of the individual sensor datasets 414 is descriptive of pressure changes detected over time by the individual sensors due to motions of a subject lying on the sensor array.

The memory 404 is further shown as containing a principal respiratory time signal 416 of a respiratory motion pattern, which is determined using the set of sensor data 414. The determined principal respiratory time signal 416 is a common one-dimensional respiratory time signal for the plurality of sensors.

The memory 404 is further shown as containing individual phase-shifts 418 of individual respiratory time signals comprised by the individual datasets 414 of the individual sensors.

The memory 404 is further shown as containing a set of residual sensor data 420 comprising a plurality of individual residual sensor datasets 422 with residual sensor data for the individual sensors. The set of residual sensor data 420 may result from an eliminating of the principal respiratory time signal 416 sensor-wise from the individual datasets 414 of the individual sensors using the individual phase-shifts 418 for the individual sensors.

The memory 404 is further shown as containing a principal cardiac time signal 424 of a cardiac motion pattern for the subject, which may be determined using the set of residual sensor data 420. The determined principal cardiac time signal 424 is a common one-dimensional cardiac time signal for the plurality of sensors.

The memory 404 is further shown as containing output data 426. The output data 426 may be displayed on a display and/or used for determining control instructions 440 for controlling a medical imaging system. The control instructions 440 may, e.g., define a timing for triggering an imaging data acquisition by the medical imaging system. The output data 426 comprise cardiac signal data 428 with the principal cardiac time signal 424. The cardiac signal data 428 may, e.g., further comprise a two-dimensional cardiac data representation descriptive of relations between individual cardiac time signals comprised by the individual residual datasets 422 of the individual sensors. The two-dimensional cardiac data representation may, e.g., comprise one or more of the following: a cardiac signal correlation map, a cardiac signal covariance map, a cardiac signal phase-shift map. The output data 426 may, e.g., further comprise respiratory signal data 430. The respiratory signal data 430 may, e.g., comprise the principal respiratory time signal 416. The respiratory signal data 430 may, e.g., comprise a two-dimensional respiratory data representation descriptive of relations between individual respiratory time signals comprised by the individual datasets 414 of the individual sensors. The two-dimensional respiratory data representation may, e.g., comprise one or more of the following: a respiratory signal correlation map, a respiratory signal covariance map, a respiratory signal phase-shift map. The output data 426 may, e.g., comprise the cardiac signal data 428 and the respiratory signal data 430 in a combined form, e.g., in form of a two-dimensional respiratory-cardiac data representation descriptive of relations between individual respiratory time signals comprised by the individual datasets of the individual sensors and the individual cardiac time signals comprised by the individual residual datasets of the individual sensors. The two-dimensional respiratory-cardiac data representation may, e.g., comprise one or more of the following: a respiratory-cardiac signal correlation map, a respiratory-cardiac signal covariance map, a respiratory-cardiac signal phase-shift map.

Fig. 15 shows a schematic block diagram of an exemplary medical system 150. The medical system 150 comprises a computational system 400 for extracting cardiac signal data from a set of sensor data acquired using a plurality of pressure sensors of a two-dimensional sensor array 100. The set of sensor data comprises a plurality of individual sensor datasets with sensor data acquired by the individual sensors of the sensor array 100. The sensor data of the individual sensor datasets is descriptive of pressure changes detected over time by the individual sensors due to motions of a subject lying on the sensor array 100. The computational system 400 may, e.g., be configured to perform the method of any of the examples disclosed herein.

The medical system 150, e.g., further comprises the two-dimensional sensor array 100. The medical system 150, e.g., further comprises a medical imaging system 110, e.g., a CT or MRI system. For example, the computational system 400 is further configured for controlling the medical imaging system 150. The computational system 400 may, e.g., be configured to use an output cardiac signal data determined using the sensor array 100 for controlling an imaging data acquisition using the medical imaging system 110.

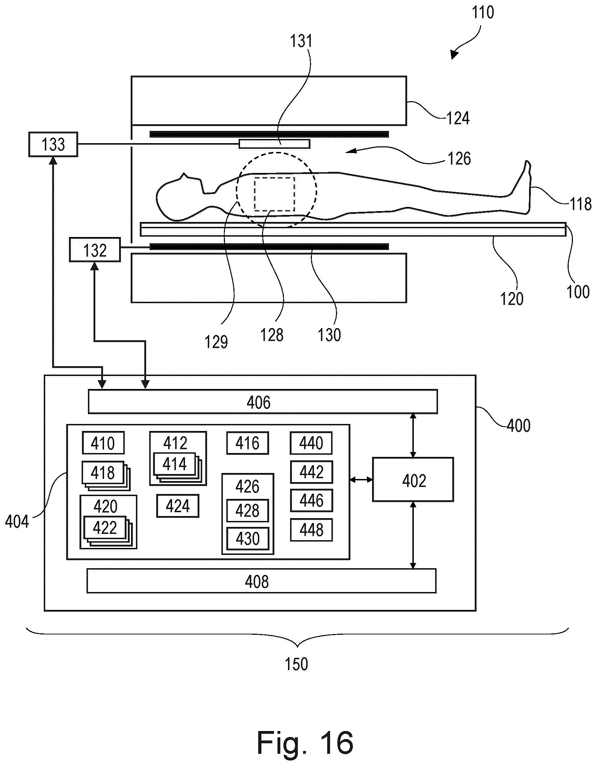

Fig. 16 shows a further exemplary medical system 150. The medical system 150 comprises a computational system 400 as shown in Figs. 14. Furthermore, the medical system 150 comprises a two-dimensional sensor array 100 with a plurality of pressure sensors. The pressure sensors of the two-dimensional sensor array 100 are configured for detecting pressure changes over time due to motions of a subject 118 lying on sensor array 100. The two-dimensional sensor array 100 may, e.g., be a sensor array as shown in Fig. 8. The sensor array 100 may, e.g., be embedded into a mattress.

Furthermore, the medical system 150 comprises a medical imaging system 110 in form of an MRI or magnetic resonance imaging system. The MRI system 110 is shown as being controlled by the computational system 400. The hardware interface 406 allows a computational component 402 of the computational system 400, e.g., a processor, to exchange data with and/or control the MRI system 110. The memory 404 of the computer system 400 is shown as containing an additional pulse sequence 442 for controlling an MRI data acquisition by the MRI system 110. The pulse sequence 442 may, e.g., be used by the computational system 400 to control the MRI system 110 to acquire imaging data 446 in form of MRI data. Memory 404 is further shown as containing the imaging data 446 acquired by controlling the MRI system 110 with the pulse sequence 442. For controlling the MRI system 110 with the pulse sequence 442, e.g., control instructions 440 may be used. The control instructions 440 may, e.g., define a timing for triggering an imaging data acquisition by the MRI system 110 controlled with the pulse sequence 442.

The MRI system 110 may, e.g., comprise a magnet 124. Magnet 124 is a superconducting cylindrical type of magnet with a bore 126 through it. The use of different types of magnets is also possible. For instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within bore 126 of the cylindrical magnet 124 there is an imaging zone 129, where the magnetic field is strong and uniform enough to perform magnetic resonance imaging. A field of view 128 is shown within imaging zone 129. The MRI data 444 is typically acquired for the field of view 128. A subject 118 is shown as being supported by a subject support 120 such that at least a portion of subject 118 is within the imaging zone 129 and the field of view 128. On the subject support 120 is the two-dimensional sensor array 100 arranged for detecting pressure changes over time due to motions of a subject 118 lying on the sensor array 100.

Within the bore 126 of the magnet there is also a set of magnetic field gradient coils 130 which is used to spatially encode magnetic spins within the bore 126 of the magnet 124. The magnetic field gradient coils 130 are connected to a gradient controller, i.e., a magnetic field gradient coil power supply 132. The magnetic field gradient coils 130 are intended to be representative. Typically, magnetic field gradient coils 130 contain three separate sets of coils for spatial encoding in three orthogonal spatial directions. The magnetic field gradient power supply 132 supplies current to the magnetic field gradient coils 130. The current supplied to the magnetic field gradient coils 130 is controlled as a function of time and may be ramped or pulsed.

Adjacent to the imaging zone 129 is a radio-frequency antenna 131 arranged for manipulating the orientations of magnetic spins within the imaging zone 129 and for receiving radio transmissions from spins also within the imaging zone 129. The radio-frequency antenna 131 may contain multiple coil elements. The radio-frequency antenna 131 is connected to a radio-frequency transceiver 133. The radio-frequency antenna 131 and radio- frequency transceiver 133 may be replaced by separate transmit and receive coils and a separate transmitter and receiver. It is understood that the radio-frequency antenna 131 and the radio-frequency transceiver 133 are representative. The radio-frequency antenna 131 is intended to also represent a dedicated transmit antenna and a dedicated receive antenna. Likewise, the transceiver 133 may also represent a separate transmitter and receivers. The radio-frequency antenna 131 may also have multiple receive/transmit elements and the radio- frequency transceiver 133 may have multiple receive/transmit channels. The transceiver 133 and the gradient controller 132 are shown as being connected to the hardware interface 406 of the computational system 400.

The computational system 400 may, e.g., use the plus sequence 442 and the control instructions 440 to control an acquisition of imaging data 444, i.e., MRI data, using the MRI system 110. The acquired MRI data may, e.g., be used to reconstruct MRI images 446.

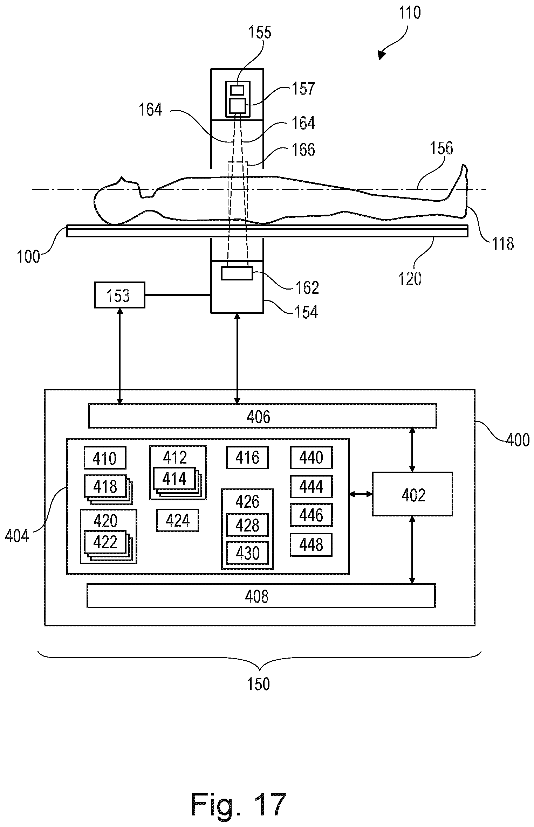

Fig. 17 shows a further exemplary medical system 150. The medical system 150 comprises a computational system 400 as shown in Figs. 14. Furthermore, the medical system 150 comprises a two-dimensional sensor array 100 with a plurality of pressure sensors. The pressure sensors of the two-dimensional sensor array 100 are configured for detecting pressure changes over time due to motions of a subject 118 lying on sensor array 100. The two-dimensional sensor array 100 may, e.g., be a sensor array as shown in Fig. 8. The sensor array 100 may, e.g., be embedded into a mattress.

Furthermore, the medical system 150 comprises a medical imaging system 110 in form of a CT or computer tomography system. The CT system 110 is shown as being controlled by the computational system 400. The hardware interface 406 allows a computational component 402 of the computational system 400, e.g., a processor, to exchange data with and/or control the CT system 110. The memory 404 of the computer system 400 is shown as containing an additional control sequence 444, i.e., CT system control commands, for controlling the CT system 110. The control sequence 444 may, e.g., be used by the computational system 400 to control the CT system 110 to acquire imaging data 446 in form of CT data. Memory 404 is further shown as containing the imaging data 446 acquired by controlling the CT system 110 with the control sequence 444. For the controlling the CT system 110 with the control sequence 444, e.g., control instructions 440 may be used. The control instructions 440 may, e.g., define a timing for triggering an imaging data acquisition by the CT system 110 controlled with the control sequence 444.

The CT system 110 may, e.g., comprise a rotating gantry 154. The gantry 154 rotates about an axis of rotation 156. There is a subject 118 lying on a subject support 120. On the subject support 120 is the two-dimensional sensor array 100 arranged for detecting pressure changes over time due to motions of a subject 118 lying on the sensor array 100. Within gantry 154 is an X-ray tube 157 placed, e.g., within an X-ray tube high voltage isolation tank. In addition, a voltage stabilizer circuit 155 may, e.g., be provided with the X-ray tube 157, within an X-ray power supply 153 or external to both. The X-ray power supply 153 supplies the X-ray tube 157 with power.

X-ray tube 157 produces X-rays 164 that pass through the subject 118 and are received by a detector 162. Within the area of box 166 is an imaging zone, from where CT imaging data 446 of the subject 118 can be acquired. This imaging data 446 may be used to reconstruct CT images 446.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer-readable medium(s) having computer executable code embodied thereon.

Any combination of one or more computer-readable medium(s) may be utilized. The computer-readable medium may be a computer-readable signal medium or a computer-readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer-readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer-readable-storage medium also refers to various types of recording media capable of being accessed by the computer device via a network or communication link. For example, data may be retrieved over a modem, over the internet, or over a local area network. Computer executable code embodied on a computer-readable medium may be transmitted using any appropriate medium, including but not limited to wireless, wire line, optical fiber cable, RF, etc., or any suitable combination of the foregoing.

A computer-readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine-executable instruction or computer executable code. References to the computational system comprising the example of a `computational system' should be interpreted as possibly containing more than one computational component or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine-executable code or instructions may be executed by multiple computational systems or processors that may be within the same computing device or which may even be distributed across multiple computing devices.

Machine-executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++, or the like and conventional procedural programming languages, such as the "C" programming language or similar programming languages and compiled into machine-executable instructions. In some instances, the computer executable code may be in the form of a high-level language or in a pre-compiled form and be used in conjunction with an interpreter which generates the machine-executable instructions on the fly. In other instances, the machine-executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine-executable instructions or computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The machine-executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode Ray Tube (CRT), storage tube, bi-stable display, electronic paper, vector display, flat panel display, Vacuum Fluorescent (VF) display, Light-Emitting Diode (LED) displays, Electroluminescent display (ELD), Plasma Display Panels (PDP), Liquid Crystal Display (LCD), Organic Light-Emitting Diode (OLED) displays, a projector, and head-mounted display.

Magnetic resonance imaging data is defined herein as being recorded measurements of RF signals emitted by atomic spins using the antenna of a magnetic resonance apparatus, like a magnetic resonance imaging system. A magnetic resonance image is defined herein as being the reconstructed two- or three-dimensional visualization of anatomic data comprises by the magnetic resonance imaging data.

Computed tomography imaging data is defined herein as being the recorded X-ray measurements taken from a variety of angles. A rotating X-ray tube and a row of detectors placed in a gantry to measure X-ray attenuations by different tissues inside a subject may be used for executing the X-ray measurements from a variety of angles. A computed tomography image is defined herein as being the reconstructed two- or three-dimensional visualization of anatomic data comprised by the computed tomography imaging data.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to an advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: sensor array
- 102: pressure sensor
- 104: mattress
- 110: medical imaging system
- 118: subject
- 120: support
- 124: magnet
- 126: bore of magnet
- 128: field of view
- 129: imaging zone
- 130: magnetic field gradient coils
- 131: radio-frequency antenna
- 132: gradient controller
- 133: radio-frequency transceiver
- 150: medical system
- 153: X-ray power supply
- 154: gantry
- 155: voltage stabilizer circuit
- 156: axis of rotation
- 157: X-ray tube
- 162: detector
- 164: X-rays
- 166: imaging zone
- 200: receiving set of sensor data
- 202: determining principal respiratory time signal
- 204: determining sensor-wise individual phase-shifts
- 206: determining two-dimensional respiratory data representation
- 208: eliminating sensor-wise principal respiratory time signal
- 210: determining principal cardiac time signal
- 212: determining two-dimensional cardiac data representation
- 214: determining a two-dimensional respiratory-cardiac data representation
- 216: outputting cardiac signal data
- 218: outputting respiratory signal data
- 220: outputting cardiac and respiratory signal data
- 230: filtering of sensor data
- 234: averaging a pair of individual respiratory time signals
- 236: determining covariances of a current averaged respiratory time signal and remaining individual respiratory time signals
- 238: selecting a remaining individual respiratory time signal
- 240: termination criterion satisfied?
- 242: averaging the current averaged respiratory time signal and the selected remaining individual respiratory time signal
- 244: phase-correcting the principal respiratory time signal
- 260: filtering of the residual sensor data
- 262: averaging the individual residual sensor data over a plurality of adjacent time windows comprised by the individual residual sensor datasets
- 264: cleansing the individual residual sensor data
- 266: averaging over the individual residual sensor data comprised by the individual residual sensor datasets
- 268: neglecting contribution of individual residual sensor datasets for the averaging
- 270: neglecting results in an improving of a peak-to-background-ratio?
- 272: removing the respective contribution from the determined principal cardiac time signal
- 274: last contribution of individual residual sensor datasets
- 276: ending refining
- 300: correlation map
- 302: section with positive correlation
- 304: section with negative correlation
- 310: covariance map
- 312: section with positive covariance
- 314: section with negative covariance
- 320: phase-shift map
- 322: section with positive phase-shift
- 324: section with negative phase-shift
- 330: map with combined correlation and covariance data
- 332: section of highest breathing motion amplitude
- 340: principal respiratory time signal
- 342: smoothened principal respiratory time signal
- 400: computational system
- 402: computational component
- 404: memory
- 406: hardware interface
- 408: user interface
- 410: machine readable instructions
- 412: set of sensor data
- 414: individual sensor datasets
- 416: principal respiratory time signal
- 418: individual phase-shifts
- 420: set of residual sensor data
- 422: individual residual sensor datasets
- 424: principal cardiac time signal
- 426: output data
- 428: cardiac signal data
- 430: respiratory signal data
- 432: imaging control data
- 440: control instructions
- 442: pulse sequence
- 444: control sequence
- 446: imaging data
- 448: reconstructed images

## Claims

1. A method for extracting cardiac signal data (428) from a set of sensor data (412) acquired using a plurality of pressure sensors (102) of a two-dimensional sensor array (100), the set of sensor data (412) comprising a plurality of individual sensor datasets (414) with sensor data acquired by the individual sensors (102) of the sensor array (100), the sensor data of the individual sensor datasets (414) being descriptive of pressure changes detected over time by the individual sensors (102) due to motions of a subject (118) lying on the sensor array (100),
the method comprising:
- receiving (200) the set of sensor data (412);
- determining (202) a principal respiratory time signal (342; 416) of a respiratory motion pattern for the subject (118) using the set of sensor data (412), the determined principal respiratory time signal (342; 416) being a common one-dimensional respiratory time signal for the plurality of sensors (102);
- determining (204) sensor-wise individual phase-shifts (418) of individual respiratory time signals comprised by the individual datasets of the individual sensors (102);
- eliminating (208) the determined principal respiratory time signal (342; 416) sensor-wise from the individual datasets of the individual sensors (102) using the determined individual phase-shifts (418) for the individual sensors (102), the eliminating resulting in a set of residual sensor data (420) comprising a plurality of individual residual sensor datasets (422) with residual sensor data for the individual sensors (102);
- determining (210) a principal cardiac time signal (424) of a cardiac motion pattern for the subject (118) using the set of residual sensor data (420), the determined principal cardiac time signal (424) being a common one-dimensional cardiac time signal for the plurality of sensors (102); and
- outputting (216) cardiac signal data (428) comprising the principal cardiac time signal (424).

2. The method of claim 1, the determining of the principal respiratory time signal (342; 416) further comprising a filtering (230) of the sensor data comprised by the set of sensor data (412), the filtering comprising a selecting of signal data from a predefined first frequency range of respiratory signal frequencies.

3. The method of any of the previous claims, the determining of the principal respiratory time signal (342; 416) further comprising one of the following:
- averaging over the individual respiratory time signals comprised by the individual datasets of the individual sensors (102);
- determining an eigenvector corresponding to a largest eigenvalue of the individual respiratory time signals comprised by the individual datasets of the individual sensors (102);
- successive averaging of selected individual respiratory time signals comprised by the individual datasets of the individual sensors (102) using a covariance analysis for the selecting, the successive averaging starting with averaging (234) of a pair of individual respiratory time signals, for which a highest covariance compared to all other pairs of individual respiratory time signals is determined,
each further step of the successive averaging comprising, until a termination criterion is satisfied:
- determining (236) covariances of a current averaged respiratory time signal and remaining individual respiratory time signals;
- selecting (238) a remaining individual respiratory time signal, for which a highest covariance with respect to the current averaged respiratory time signal is determined compared to all other remaining individual respiratory time signals;
- averaging (242) the current averaged respiratory time signal and the selected remaining individual respiratory time signal.

4. The method of any of the previous claims, the determining of the principal respiratory time signal (342; 416) further comprising one of the following:
- a smoothening of the principal respiratory time signal (340);
- a phase-correction (244) for the principal respiratory time signal (342; 416), the phase correction comprising:
- using the determined sensor-wise individual phase-shifts (418) of individual respiratory time signals to determine phase-corrected individual respiratory time signals,
- using the phase-correction individual respiratory time signals for updating the principal respiratory time signal (342; 416) with a phase-corrected principal respiratory time signal to be used for the eliminating.

5. The method of any of the previous claims, the determining of the principal cardiac time signal (424) further comprising a filtering (260) of the residual sensor data comprised by the set of residual sensor data (420), the filtering comprising a selecting of residual signal data from a predefined second frequency range of cardiac signal frequencies.

6. The method of any of the previous claims, the determining of the principal cardiac time signal (424) further comprising one or more of the following:
- an averaging (262) of the individual residual sensor data over a plurality of adjacent time windows comprised by the individual residual sensor datasets (422);
- a cleansing (264) of the individual residual sensor data comprised by the individual residual sensor datasets (422), the cleansing comprising subtracting of one or more contributions to the individual residual sensor data, which are negligible for the determining of the principal cardiac time signal (424).

7. The method of any of the previous claims, the determining of the principal cardiac time signal (424) further comprising averaging (266) over the individual residual sensor data comprised by the individual residual sensor datasets (422), the averaging in particular being executed in Fourier space by determining Fourier transforms using the individual residual sensor data and averaging the resulting Fourier transforms.

8. The method of claim 7, the determining of the principal cardiac time signal (424) further comprising a refining of the principal cardiac time signal (424), the refining of the principal cardiac time signal (424) comprising:
- successively (268) neglecting contributions of individual residual sensor datasets (422) for the averaging;
- checking (270), whether the neglecting results in an improving of a peak-to-background-ratio; and,
- in response to determining an improving, removing (272) the respective contribution from the determined principal cardiac time signal (424).

9. The method of any of the previous claims, the method further comprising:
- determining (212) a two-dimensional cardiac data representation descriptive of relations between individual cardiac time signals comprised by the individual residual datasets of the individual sensors (102), the two-dimensional cardiac data representation in particular comprising one or more of the following: a cardiac signal correlation map, a cardiac signal covariance map, a cardiac signal phase-shift map;
- the output cardiac signal data (428) further comprising the two-dimensional cardiac data representation.

10. The method of any of the previous claims, the method further comprising outputting (218) the principal respiratory time signal (342; 416).

11. The method of any of the previous claims, the method further comprising:
- determining (206) a two-dimensional respiratory data representation descriptive of relations between individual respiratory time signals comprised by the individual datasets of the individual sensors (102), the two-dimensional respiratory data representation in particular comprising one or more of the following: a respiratory signal correlation map (300), a respiratory signal covariance map (310), a respiratory signal phase-shift map (320);
- outputting (218) the two-dimensional respiratory data representation.

12. The method of any of the previous claims, the method further comprising:
- determining (214) a two-dimensional respiratory-cardiac data representation descriptive of relations between individual respiratory time signals comprised by the individual datasets of the individual sensors (102) and the individual cardiac time signals comprised by the individual residual datasets of the individual sensors (102);
- outputting (220) the two-dimensional respiratory-cardiac data representation.

13. A computer program for extracting cardiac signal data (428) from a set of sensor data (412) acquired using a plurality of pressure sensors (102) of a two-dimensional sensor array (100), the set of sensor data (412) comprising a plurality of individual sensor datasets (414) with sensor data acquired by the individual sensors (102) of the sensor array (100), the sensor data of the individual sensor datasets (414) being descriptive of pressure changes detected over time by the individual sensors (102) due to motions of a subject (118) lying on the sensor array (100),
the computer program comprising machine executable instructions (410) for execution by a computational system (400), wherein execution of the machine executable instructions (410) causes the computational system (400) to perform the method of any of the previous claims.

14. A medical system (150) comprising a computational system (400) for extracting cardiac signal data (428) from a set of sensor data (412) acquired using a plurality of pressure sensors (102) of a two-dimensional sensor array (100), the set of sensor data (412) comprising a plurality of individual sensor datasets (414) with sensor data acquired by the individual sensors (102) of the sensor array (100), the sensor data of the individual sensor datasets (414) being descriptive of pressure changes detected over time by the individual sensors (102) due to motions of a subject (118) lying on the sensor array (100),
the computational system (400) comprising a memory (404) storing machine executable instructions (410), wherein execution of the machine executable instructions (410) causes the computational system (400) to perform the method of any of the previous claims 1 to 12.

15. The medical system (150) of claim 14, the medical system (150) further comprising one or more of the following:
- the two-dimensional sensor array (100);
- a medical imaging system (110), the machine executable instructions (410) further being configured for controlling the medical imaging system (110), wherein the execution of the machine executable instructions (410) further causes the computational system (400) to use the output cardiac signal data (428) for controlling an imaging data acquisition using the medical imaging system (110).
